# EUROPEAN PATENT SPECIFICATION

(11) **EP 2 448 962 B1**
(45) Date of publication and mention of the grant of the patent: **19.10.2016**
(21) Application number: 10726515.9
(22) Date of filing: 29.06.2010
(51) Int. Cl.: C07K 14/62, A61P 3/10

(54) **INSULIN DERIVATIVES**
INSULIN-DERIVATE
DÉRIVÉS D'INSULINE

(30) Priority: 30.06.2009 EP 09164103; 19.08.2009 US 235112 P
(43) Date of publication of application: 09.05.2012
(73) Proprietor: Novo Nordisk A/S, 2880 Bagsværd (DK)
(72) Inventor: HOEG-JENSEN, Thomas, DK-2880 Bagsværd (DK); JAKOBSEN, Palle, DK-2880 Bagsværd (DK); SENSFUSS, Ulrich, DK-2880 Bagsværd (DK); FLEDELIUS, Christian, DK-2880 Bagsværd (DK); RIBEL-MADSEN, Ulla, DK-2880 Bagsværd (DK)
(86) International application number: PCT/EP2010/059194
(87) International publication number: WO 2011/000823

(56) References cited:
- WO-A1-01/92334
- WO-A2-03/048195

## Description

### FIELD OF THIS INVENTION

The present invention relates to novel insulin derivatives which are useful in the treatment of diabetes and related aspects.

### BACKGROUND OF THIS INVENTION

In man, insulin lowers blood glucose and is used for treatment of diabetes type 1 and type 2, with the goal of adjusting blood glucose towards healthy levels. In healthy persons, blood glucose levels are regulated close to 5 mM during the fasting state, whereas values up towards 10 mM can occur for a few hours after a meal. Blood glucose levels are influenced by many factors such as timing and character of meals and insulin administrations, exercise, infections and more. Blood glucose can fluctuate widely and unpredictably in diabetes patients (for example, in the range 1-30 mM). It could therefore be helpful if an administered insulin drug could auto-adjust its activity or availability to suit the blood glucose level at any given time. Specifically, such a glucose-sensitive insulin should have low or no activity or availability during situations of low blood glucose (below about 3 mM, i.e., hypoglycemia), and high activity or availability in response to high blood glucose (above about 10 mM, i.e., hyperglycémie).

Glucose-modulated insulin release from subcutaneous insulin depots have been pursued by several principles, but subcutaneous depots generally suffer from problems of lag (delay) of compound distributions between tissue and the blood stream. The lag of glucose fluctuations from blood to subcutis is approximately 15 minutes, and the lag of insulin drug from release in subcutis to appearance in the circulation is in the range of ½-2 hours. Discovery of methods for formation of glucose-sensitive insulin as circulating depots could be advantageous because the lag to and from subcutis would be eliminated. Reversible binding to circulating proteins such as serum albumin can help prolonging the *in vivo* activity of drugs. Albumin binding as a protraction principle has been exploited for insulin and other peptides by conjugation of the drug with fatty acids, fatty diacids or related compounds, optionally incorporated via various linkers.

According to the title, US 5,478,575 relates to polymers having benzeneboronic acid groups and insulin complexes of same of a sugar response type.

According to claims 1, WO 01/92334 (Novo Nordisk A/S) relates to an insulin derivative containing a glucose-sensing group.

According to claims 1, WO 03/048195 (Novo Nordisk A/S) relates to an insulin derivative comprising a glucose-sensing group and a polyol moiety.

### OBJECTS OF THIS INVENTION

An aspect of this invention relates to the furnishing of insulin derivatives which, after administration, delivers insulin as a function of the glucose concentration in the tissue.

An aspect of this invention relates to the furnishing of insulin derivatives having low or no activity/availability during situations of low blood glucose levels, for example at levels below about 3 mM glucose.

Another aspect of this invention relates to the furnishing of insulin derivatives having high activity/availability in response to high blood glucose levels, for example, above about 10 mM glucose.

Another aspect of this invention relates to the furnishing of methods for formation of glucose-sensitive insulin as circulating depots.

Another aspect of this invention relates to the furnishing of insulin derivatives having glucose-sensitive albumin binding.

Another aspect of this invention relates to the furnishing of glucose-based insulin activity and/or release modulated in the circulation/blood steam.

The object of this invention is to overcome or ameliorate at least one of the disadvantages of the prior art, or to provide a useful alternative.

### DEFINITIONS

The term "human insulin" as used herein means the human insulin hormone whose structure and properties are well-known. Human insulin has two polypeptide chains, named the A-chain and the B-chain consisting of 21 and 30 amino acids, respectively.

The term "insulin" or natural insulin as used herein covers human insulin and insulins of other species than human.

The term "insulin analogue" as used herein covers a modified insulin wherein one or more amino acid residues have been substituted (exchanged) by other amino acid residues and/or wherein one or more amino acid residues have been deleted from the insulin and/or wherein one or more amino acid residues have been added and/or inserted to the insulin.

In one embodiment an insulin analogue comprises less than 8 modifications (substitutions, deletions, additions (including insertions) and any combination thereof) relative to the parent insulin, alternatively less than 7 modifications relative to the parent insulin, alternatively less than 6 modifications relative to the parent insulin, alternatively less than 5 modifications relative to the parent insulin, alternatively less than 4 modifications relative to the parent insulin, alternatively less than 3 modifications relative to the parent insulin, alternatively less than 2 modifications relative to the parent insulin.

Modifications in the insulin molecule are denoted stating the chain (A or B), the position and the one or three letter code for the amino acid residue substituting the native amino acid residue.

By "desB30" is meant natural insulin or an analogue thereof lacking the B30 amino acid residue.

An insulin derivative is insulin carrying a group different from the natural amino acid residues and insulin analogues carrying a group different from the natural amino acid residues.

An amino acid residue is an amino acid from which a hydrogen atom has been removed from an amino group and/or a hydroxy group has been removed from a carboxy group.

Herein, an insulin residue or an insulin analogue residue is insulin or an insulin analogue wherein a hydrogen atom has been removed from one or two amino groups.

The term alkyl as used herein covers a straight or branched alkyl group, preferably containing 1-8 carbon atoms, more preferred 1-4 carbon atoms, such as methyl, ethyl, propyl and isopropyl. Herein, the term "C₁-C₃-alkyl" covers an alkyl group with 1 through 3 carbon atoms such as methyl, ethyl, propyl and isopropyl.

An alkylene group or moiety is the divalent moiety corresponding to an alkyl group. Hence, the term "C₁-C₃-alkylene" covers an alkylene group with 1 through 3 carbon atoms such as methylene, ethylene, propylene and isopropylene.

The term alkanoyl group (or acyl group) is a group derived by the removal of a hydroxy group from a carboxylic group. An alkanoyl group can be illustrated by the general formula R'-CO- (or R'C (=O)-) wherein R' is an alkyl group. The alkyl group present in the alkanoyl group is a straight or branched alkyl group which may contain 6-22 carbon atoms, preferably 10-20 carbon atoms.

### SUMMARY OF THE INVENTION

This invention relates to the compounds covered by the claims below.

### BRIEF DESCRIPTION OF THE FIGURES

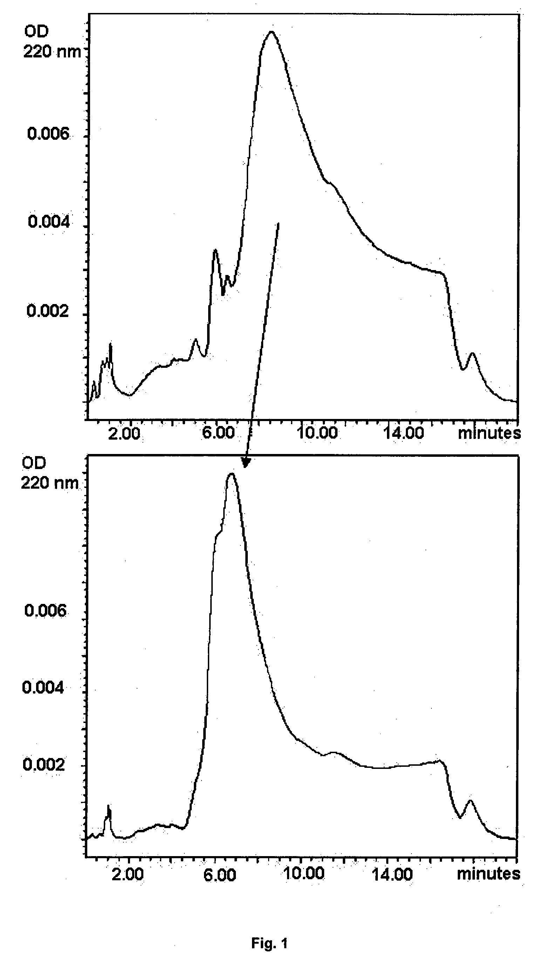

Figure 1 shows the chromatographic profile of the compound of example 2 eluted from albumin column using buffer with no glucose and 50 mM glucose buffer (phosphate buffered saline, pH 7.4).

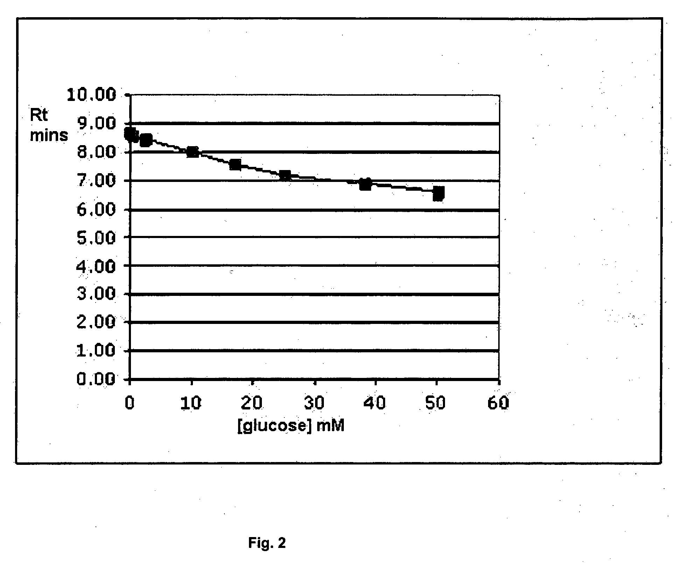

Figure 2 shows the shift in retention times of elution of the compound of example 2 from albumin column as a function of glucose concentrations in the range 0-50 mM (duplicate experiments, series 1 and 2).
Figure 3 shows the pharmacokinetic profiles after i.v. administration of insulin of example 22 (i.e., a compound according to this invention), insulin of example 24 (i.e., a compound with no-B(OH)₂ group) or vehicle in fed anaesthetized Wistar rats, *vide* example 30.
Figure 4 shows the pharmacokinetic profiles after i.v. administration of insulin of example 22 or 24 or vehicle in fed anaesthetized Wistar rats (log insulins), *vide* example 30.
Figure 5 shows the pharmacokinetic profiles after i.v. administration of insulin of example 22 or 24 or vehicle in fed anaesthetized ZDF rats, *vide* example 30.
Figure 6 shows the pharmacokinetic profiles after i.v. administration of insulin of example 22 or 24 or vehicle in fed anaesthetized ZDF rats (log insulins), *vide* example 30.

### DETAILED DESCRIPTION OF THIS INVENTION

As appears from claim 1, in the novel insulin derivatives, the insulin moiety (designated Ins) is an insulin residue or an insulin analogue residue. Said insulin analogue can be an insulin analogue having the natural lysine residue in the B29 position or an insulin analogue having a lysine residue connected to the C terminal end of the A chain, i.e., a A22Lys insulin analogue. In the novel insulin derivatives of this invention, a moiety of the general formula -X-Y-Z and/or -X¹-Y¹-Z¹ (wherein the symbols are as mentioned below) is/are attached to an ε amino group in a Lys (K) residue in an insulin analogue.

Hence, in one aspect, this invention relates to insulin derivatives of the general formula la: Ins-X-Y-Z wherein Ins, X, Y and Z are as herein defined (and n is 1 and m is zero). In the compounds of formula la, the moiety -X-Y-Z is connected to an ε amino group present in a B29 lysine residue or an ε amino group present in a A22 lysine residue.

In another aspect, this invention relates to insulin derivatives of the general formula Ib: Z¹-Y¹-X¹-Ins-X-Y-Z wherein Ins, X, X¹, Y, Y¹, Z and Z¹ are as herein defined (and n and m are both 1). In the compounds of formula Ib, the moieties -X-Y-Z and -X¹-Y¹-Z¹ are connected to an ε amino group present in a B29 lysine residue and an ε amino group present in a A22 lysine residue.

Both in the moiety of the general formula -CO-(C₁-C₃-Alkylene)-CH(COOH)-NH- and in the moiety of the general formula -CO-(C₁-C₃-alkylene)-SO₂-NH- each of which are here illustrated by the symbols X and X¹, the amino group is connected to the Y/Y¹ moiety and, consequently, the carbonyl group thereof is connected to "Ins".

The ω-amino alkanoyl group illustrated by the symbols Y and Y¹ is a divalent group/moiety. In one embodiment, the amino group in said ω-amino alkanoyl group is connected to the group illustrated by the symbol X (or X¹) and, simultaneously, the carbonyl group in said ω-amino alkanoyl group is connected to the group illustrated by the symbol Z (or Z¹). In one embodiment, the amino group in said ω-amino alkanoyl group is connected to the group illustrated by the symbol Z (or Z¹) and, simultaneously, the carbonyl group in said ω-amino alkanoyl group is connected to the group illustrated by the symbol X (or X¹).

Building blocks incorporating fatty acid motifs as well as boronates are synthesised and conjugated to insulin and insulin analogues. The glucose-sensitive insulins can be administered by subcutaneous injection where formation of depots will contribute to prolonged activity. The activity profile of the glucose-sensitively albumin-bound insulin in the circulation will be modulated by the blood glucose concentration at any given time.

The insulin derivatives of this invention are long-acting due to albumin binding, and in some cases increased tendency to oligomerisation in the subcutaneous depot, ensuring slow diffusion to the circulation. In the circulation, the glucose-sensitive albumin binding regulates the free fraction of the insulin derivative of this invention in a glucose-dependent manner. The insulin receptor affinity of the compounds of this invention are within the desired range.

### PRODUCTION OF INSULIN ANALOGUES AND COMPOUNDS OF THIS INVENTION

The production of polypeptides, e.g., insulin analogues, is well known in the art. Insulin analogues may for instance be produced by classical peptide synthesis, e.g., solid phase peptide synthesis using t-Boc or Fmoc chemistry or other well established techniques, see, e.g., Greene and Wuts, "Protective Groups in Organic Synthesis", John Wiley & Sons, 1999. The insulin analogue may also be produced by a method which comprises culturing a host cell containing a DNA sequence encoding the analogue and capable of expressing the insulin analogue in a suitable nutrient medium under conditions permitting the expression of the insulin analogue. Several recombinant methods may be used in the production of human insulin and human insulin analogues. Three non-limiting examples of methods which may be used in the production of insulins in microorganisms such as, e.g., *Escherichia coli* and *Saccharomyces cerevisiae* are, e.g., disclosed in WO 2008/034881. For insulin analogues comprising non-natural amino acid residues, the recombinant cell should be modified such that the non-natural amino acids are incorporated into the analogue, for instance by use of tRNA mutants. Hence, briefly, the insulin analogues are prepared analogously to the preparation of known insulin analogues.

Furthermore, the compounds of this invention are prepared in a manner known *per se,* for example, analogously to the preparation of known compounds or analogously to the preparation of similar compounds.

### USE OF THE COMPOUNDS OF THIS INVENTION

The route of administration may be any route which effectively transports a compound of this invention to the desired or appropriate place in the body, such as parenterally, for example, subcutaneously, intramuscularly or intraveneously. Alternatively, a compound of this invention can be administered orally, pulmonary, rectally, transdermally, buccally, sublingually, or nasally.

For parenterally administration, a compound of this invention is formulated analogously with the formulation of known insulins. Furthermore, for parenterally administration, a compound of this invention is administered analogously with the administration of known insulins and the physicians are familiar with this procedure.

Parenteral administration can be performed by means of a syringe, optionally a pen-like syringe. Alternatively, parenteral administration can be performed by means of an infusion pump.

Injectable compositions containing a compound of this invention can be prepared using the conventional techniques of the pharmaceutical industry which involve dissolving and mixing the ingredients as appropriate to give the desired end product. Thus, according to one procedure, a compound of this invention is dissolved in an amount of water which is somewhat less than the final volume of the composition to be prepared. An isotonic agent, a preservative and a buffer is added as required and the pH value of the solution is adjusted, if necessary, using an acid, for example, hydrochloric acid, or a base, for example, aqueous sodium hydroxide, as needed. Finally, the volume of the solution is adjusted with water to give the desired concentration of the ingredients.

More precisely, an insulin preparation of this invention, for example a solution or suspension, may be prepared by dissolving a compound of this invention in an aqueous medium at slightly acidic conditions, for example, in a concentration in the range from about 240 to about 2400 nmole/ml. The aqueous medium is made isotonic, for example, with sodium chloride or glycerol. Furthermore, the aqueous medium may contain zinc ions, buffers such as acetate and citrate and preservatives such as m-cresol or phenol. The pH value of the solution is adjusted towards neutrality without getting too close to the isoelectric point of the compound of this invention in order to avoid precipitation. The pH value of the final insulin preparation depends upon which compound of this invention is used, the concentration of zinc ions and the concentration of the compound of this invention. The insulin preparation is made sterile, for example, by sterile filtration.

The insulin preparations of this invention are used similarly to the use of the known insulin preparations.

The amount of a compound of this invention to be administered, the determination of how frequently to administer a compound of this invention, and the election of which compound or compounds of this invention to administer, optionally together with another antidiabetic compound, is decided in consultation with a practitioner who is familiar with the treatment of diabetes.

### PREFERRED FEATURES OF THIS INVENTION

To sum up and supplement the above statements, the features of this invention are as follows:
1. Insulin derivatives having the general formula I:

   (Z¹-Y¹-X¹)ₘ-Ins-(X-Y-Z)ₙ (I)

   wherein n is zero or 1; m is zero or 1; with the proviso that not both n and m are zero;
   Ins represents insulin or an insulin analogue as defined herein from which a hydrogen atom has been removed from an ε amino group present in a B29 lysine residue or present in a A22 lysine residue and wherein the amino group present in the N terminal amino acid residue in the A and/or B chain, optionally, is substituted by one or two alkyl groups (preferably methyl or ethyl), wherein the group(s) of the general formula -X-Y-Z and/or -X¹-Y¹-Z¹ is/are attached to an ωε amino group in a Lys (K) residue in the insulin molecule;
   X and X¹, independently of each other, is a bond, a moiety of the general formula -CO-(C₁-C₃-alkylene)-CH(COOH)-NH- wherein the amino group is connected to the Y/Y¹ moiety or a moiety of the general formula -CO-(C₁-C₃-alkylene)-SO₂-NH- wherein the amino group is connected to the Y/Y¹ moiety;
   Y and Y¹, independently of each other, is a ω-amino alkanoyl group containing 6 to 22 carbon atoms and wherein a hydrogen atom has been removed from the ω-amino group; and
   Z and Z¹, independently of each other, is a group of one of the following two, general formulae

   -SO₂-Ar or -CO-Ar,

   wherein Ar is a phenyl group substituted by one or more -B(OH)₂ groups and, optionally, substituted by one or more halogen atoms.
2. A compound according to the preceding clause wherein the moiety designated -CO-(C₁-C₃-alkylene)-CH(COOH)-NH- for the symbol X and/or X¹ is a γ-glutamyl moiety having the formula-CO-CH₂-CH₂-CH(COOH)-NH-.
3. A compound according to any one of the preceding clauses wherein X and X¹ are the same and both are a bond.
4. A compound according to any one of the preceding clauses wherein one of X and X¹ is a bond,
5. A compound according to any one of the preceding clauses to the extent possible, wherein X and X¹ are the same and both are a moiety of the general formula -CO-(C₁-C₃-alkylene)-CH(COOH)-NH-
6. A compound according to any one of the preceding clauses to the extent possible, e.g. clause 1,
   wherein X and X¹ are the same and both are a γ-glutamyl group.
7. A compound according to any one of the preceding clauses to the extent possible, wherein X or X¹ are the same and both are a moiety of the general formula -CO-(C₁-C₃-alkylene)-CH(COOH)-NH-
8. A compound according to any one of the preceding clauses to the extent possible wherein X or X¹ is a γ-glutamyl group.
9. A compound according to any one of the preceding clauses to the extent possible, e.g. clause 1,
   wherein X and X¹ are the same and both are a butyroylsulfonylamine group (-CO-CH₂-CH₂-CH₂-SO₂-NH-) wherein the amino group is connected to the Y/Y¹ moiety.
10. A compound according to any one of the preceding clauses wherein the ω-amino alkanoyl group (Y and/or Y¹) contains 10 to 20 carbon atoms.
11. A compound according to any one of the preceding clauses wherein the ω-amino alkanoyl group (Y and/or Y¹) contains 12 to 18 carbon atoms.
12. A compound according to any one of the preceding clauses wherein the ω-amino alkanoyl group (Y and/or Y¹) contains 13 to 17 carbon atoms.
13. A compound according to any one of the preceding clauses to the extent possible, wherein the alkyl moiety of the ω-amino alkanoyl group (Y and/or Y¹) is a straight alkyl group.
14. A compound according to any one of the preceding clauses to the extent possible, wherein the amino group in the ω-amino alkanoyl group (Y and/or Y¹) is connected to Z and/or Z¹, and the carbonyl group of the ω-amino alkanoyl group (Y and/or Y¹) is connected to X and/or X¹.
15. A compound according to any one of the preceding clauses to the extent possible wherein Ar present in Z and/or Z¹ carries one or two -B(OH)₂ groups.
16. A compound according to any one of the preceding clauses, to the extent possible, wherein Ar present in Z and/or Z¹ carries only one -B(OH)₂ group.
17. A compound according to any one of the preceding clauses, to the extent possible, wherein Ar present in Z and/or Z¹ carries only one -B(OH)₂ group which is in the meta or para position in relation to the -SO₂- or -CO- moiety of the -SO₂-Ar or -CO-Ar moiety, respectively.
18. A compound according to any one of the preceding clauses, to the extent possible, wherein Ar present in Z and/or Z¹ carries two -B(OH)₂ groups which are in the meta position in relation to the-SO₂- or -CO- moiety of the -SO₂-Ar or -CO-Ar moiety, respectively.
19. A compound according to any one of the preceding clauses to the extent possible wherein Ar present in Z and/or Z¹ carries one or two halogen atoms.
20. A compound according to the preceding clause to the extent possible wherein Ar present in Z and/or Z¹ carries one or two fluoro atoms.
21. A compound according to the preceding clause to the extent possible, wherein Ar present in Z and/or Z¹ carries one or two chloro atoms.
22. A compound according to any one of the preceding, possible clauses wherein Z and/or Z¹ is elected from the group consisting of the following groups (where the dotted line is the point of attachment): 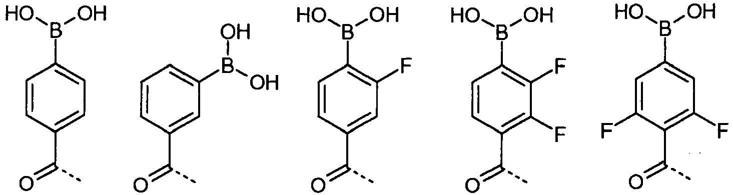 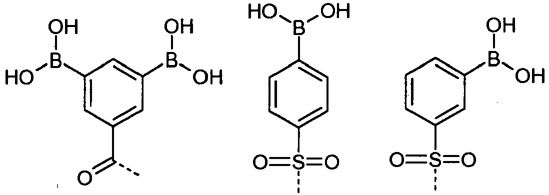
23. A compound according to any one of the preceding clauses to the extent possible, wherein m and n are each 1 and the groups of the general formula -X-Y-Z and -X¹-Y¹-Z¹ are identical.
24. A compound according to any one of the preceding clauses to the extent possible wherein Ins is human insulin, a natural insulin of other species than human or an analogue of human insulin wherein one or more amino acid residues have been substituted (exchanged) by other amino acid residues, wherein one or more amino acid residues have been deleted from the insulin and/or wherein one or more amino acid residues have been added and/or inserted and, preferably, said insulin analogue comprises less than 8 modifications (substitutions, deletions, additions (including insertions) and any combination thereof) relative to human insulin, alternatively less than 7 modifications relative to human insulin, alternatively less than 6 modifications relative to human insulin, alternatively less than 5 modifications relative to human insulin, alternatively less than 4 modifications relative to human insulin, alternatively less than 3 modifications relative to human insulin, alternatively less than 2 modifications relative to human insulin.
25. A compound according to any one of the preceding, possible clauses wherein a hydrogen atom has been removed from an ε amino group present in a B29 lysine residue and a hydrogen atom has been removed from an ε amino group present in a A22 lysine residue.
26. A compound according to any one of the preceding, possible clauses wherein the insulin residue designated "Ins" has E (Glu) in the A14 position, and/or K (Lys) in the A22 position, and/or H (His) in the B25 position, and/or R (Arg) in the B29 position and/or no amino acid residue in the B30 position.
27. A compound according to any one of the preceding, possible clauses wherein the insulin residue designated "Ins" is elected from the group of human insulin, desB30 human insulin, A14E B25H desB30 human insulin, A22K desB30 human insulin and A14E A22K B25H desB30 human insulin.
28. A compound according to any one of the preceding, possible clauses wherein the insulin residue designated "Ins" is elected from the group of A22K, B29R, desB30 human insulin and A14E, A22K, B25H, B29R, desB30 human insulin and A22K, desB30 human insulin.
29. A compound according to any one of the preceding, possible clauses wherein the insulin residue designated "Ins" is elected from the group of A22K, B29R, desB30 human insulin and A14E, A22K, B25H, B29R human insulin; A14E, A22K, B25H human insulin; A14E, B25H human insulin; A22K, B29R human insulin; and A22K human insulin.
30. A compound according to any one of the preceding clauses to the extent possible wherein n and m are each 1, and the groups designated -X-Y-Z are attached to the ε amino group in a Lys (K) residue in the A22 and B29 positions in the insulin molecule.
31. A compound according to any one of the preceding product claims to the extent possible, which is any one of the compounds mentioned specifically in the above specification such as in the specific examples, especially any one of examples 1 *et seq.* below.
32. A compound according to any one of the preceding clauses, to the extend possible, selected from the group consisting of B29*N*^{ε}-15-(4-boronobenzenesulfonylamino)pentadecanoyl desB30 human insulin; A22*N*^{ε}-16-(2,3-difluoro-4-boronobenzoylamino)hexadecanoyl B29N(epsilon)-16-(2,3-difluoro-4-boronobenzoylamino)hexadecanoyl A14E A22K B25H desB30 human insulin; B29*N*^{ε}-12-(3-boronobenzenesulfonylamino)dodecanoyl desB30 human insulin; B29*N*^{ε}-16-(2,3-difluoro-4-boronobenzoylamino)hexadecanoyl-γ-L-glutamyl desB30 human insulin; B29*N*^{ε}-16-(3,5-diboronobenzoylamino)hexadecanoyl desB30 human insulin; B29*N*^{ε}-16-(2,3-difluoro-4-boronobenzoylamino)hexadecanoyl desB30 human insulin; B29*N*^{ε}-16-(3-fluoro-4-boronobenzoylamino)hexadecanoyl desB30 human insulin; B29*N*^{ε}-16-(3-fluoro-4-boronobenzoylamino)hexadecanoyl-γ-L-glutamyl desB30 human insulin; B29*N*^{ε}-16-(2,6-difluoro-4-boronobenzoylamino)hexadecanonyl desB30 human insulin; A1(*N,N*-dimethyl), B1(*N,N-*dimethyl), B29*N*^{ε}-15-(4-boronobenzenesulfonyl-amino)pentadecanoyl desB30 human insulin; A22*N*^{ε}-16-(3,5-diboronobenzoylamino)hexadecanoyl A22K B29R desB30 human insulin; A22*N*^{ε}-16-(3,5-diboronobenzoylamino)hexadecanoyl A14E A22K B25H B29R desB30 human insulin; A22*N*^{ε}-16-(3,5-diboronobenzoylamino)hexadecanoyl-L-γ-glutamyl A14E A22K B25H B29R desB30 human insulin; B29-*N*^{ε}-{4-[16-(3-boronobenzenesulfonylamino)hexadecanoylsulfamoyl]butanoyl} desB30 human insulin; A22*N*^{ε}-15-(3,5-diboronobenzoylamino)pentadecanoyl B29*N*^{ε}-15-(3,5-diboronobenzoylamino)pentadecandioyl A14E A22K B25H desB30 human insulin; A22*N*^{ε}-15-(3,5-diboronobenzoylamino)pentadecanoyl B29*N*^{ε}-15-(3,5-diboronobenzoylamino)pentadecandioyl A22K desB30 human insulin; B29*N*^{ε}-16-(3,5-diboronobenzoylamino)hexadecanoyl-L-γ-glutamyl A14E B25H desB30 human insulin; A22*N*^{ε}-15-(3,5-diboronobenzoylamino)pentadecanoyl-γ-L-glutamyl B29*N*^{ε}-15-(3,5-diboronobenzoylamino)pentadecandioyl-γ-L-glutamyl A14E A22K B25H desB30 human insulin; B29*N*^{ε}**-**{4-[16-(3-boronobenzenesulfonylamino)hexadecanoylsulfamoyl]butanoyl} A14E B25H desB30 human insulin; A22*N*^{ε}-12-(3,5-diboronobenzoylamino)dodecanoyl B29*N*^{ε}-12-(3,5-diboronobenzoylamino)dodecandioyl A14E A22K B25H desB30 human insulin; A22*N*^{ε}-12-(3,5-diboronobenzoylamino)dodecanoyl B29*N*^{ε}-12-(3,5-diboronobenzoylamino)dodecandioyl A22K desB30 human insulin; and B29*N*^{ε}-16-(2,3-difluoro-4-boronobenzoylamino)hexadecanoyl A14E B25H des B30 human insulin.
33. A compound according to any one of the preceding clauses, to the extend possible, selected from the group consisting of A22*N*^{ε}-12-(3,5-diboronobenzoylamino)dodecanoyl-gamma-L-glutamyl B29*N*^{ε}-12-(3,5-diboronobenzoylamino)dodecandioyl-gamma-L-glutamyl A22KdesB30 human insulin; A22*N*^{ε}-12-(3,5-diboronobenzoylamino)dodecanoyl-gamma-L-glutamyl B29*N*^{ε}-12-(3,5-diboronobenzoylamino)dodecandioyl-gamma-L-glutamyl A14E A22K B25H desB30 human insulin; B29*N*^{ε}-16-(2,6-difluoro-4-boronobenzoylamino)pentadecanoyl A14E B25H desB30 human insulin; B29*N*^{ε}-16-(2,6-difluoro-4-boronobenzoylamino)hexadecanoyl A14E B25H desB30 human insulin; and B29*N*^{ε}-16-(4-boronobenzoylamino)hexadecanoyl A14E B25H desB30 human insulin.
34. A compound according to any one of the preceding clauses, to the extend possible, which is B29*N*^{ε}-16-(2,3-difluoro-4-boronobenzoylamino)hexadecanoyl A14E B25H des B30 human insulin.
35. A compound according to any one of the preceding product claims for use as a medicament or for use in a medicament.
36. A compound according to any one of the preceding product claims for treating diabetes or the use of a compound according to any one of the preceding product claims for the preparation of a medicament for the treatment of diabetes.
37. The use of a compound according to any one of the preceding product clauses for the preparation of a pharmaceutical composition.
38. The use according to the preceding clause, which composition can be used for the treatment of diabetes.

The following examples are offered by way of illustration, not by limitation.

In the examples, first the systematic name of the compound of this invention and then a chemical illustration of the chemical formula is given.

In, for example, example 1, the insulin moiety is illustrated by the expression "desB29, desB30 human insulin". However, this does not mean that there is no B29 amino acid in the compound of example 1, because the B29 amino acid residue being lysine is illustrated in the chemical moiety connected to the "desB29, desB30 human insulin" moiety.

In compounds of this invention having both a substituent of the general formula -X-Y-Z and a substituent of the general formula -X¹-Y¹-Z¹, as, for example, in example 2, it appears from the systematic name of the compound that both the A22Lys and the B29Lys amino acid residues are substituted. Even though the insulin moiety in the chemical formula is illustrated by the expression "A14E, A22K, B25H, desA22, desB29, des B30 insulin", this does not mean that there are no amino cid residue in the A22 and the B29 positions, because the A22 and the B29 amino acid residues being lysine are illustrated in the chemical moiety connected to the "A14E, A22K, B25H, desA22, desB29, des B30 insulin" moiety (notice, for example, the number "2" appearing after the parenthesis in the formula).

In the names of compounds, B29*N*^{ε} and A22*N*^{ε} indicates that the following group in the name is connected to the ε amino group of the amino acid residue present in the B29 or A22 position, respectively.

### Example 1

B29*N*^{ε}-15-(4-Boronobenzenesulfonylamino)pentadecanoyl desB30 human insulin

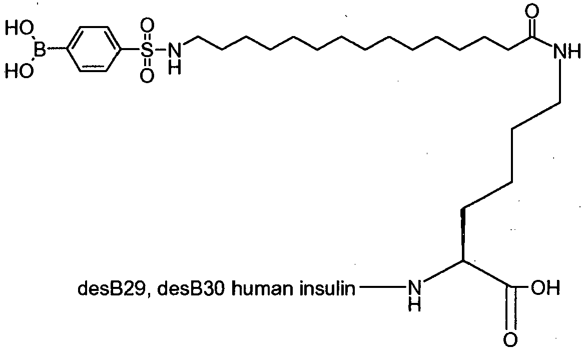

### Hexadecandioc acid mono-tert-butyl ester

Hexadecandioic acid (40.0 g, 140 mmol) was suspended in toluene (250 ml) and the mixture was heated to reflux. *N,N*-Dimethylformamide di-tert-butyl acetal (76.3 g, 375 mmol) was added drop-wise over 4 hours. The mixture was refluxed overnight. The solvent was removed *in vacuo* at 50 °C, and the crude material was suspended in dichloromethane/ethyl acetate (500 ml, 1:1) and stirred for 15 mins. The solids were collected by filtration and triturated with dichloromethane (200 ml). The filtrated were evaporated *in vacuo* to give crude mono-tert-butyl hexadecandioate, 30 grams. This material was suspended in dichloromethane (50 ml), cooled with ice for 10 mins, and filtered. The solvent was removed *in vacuo* to leave 25 gram crude mono-tert-butyl hexadecandioate, which was recrystallized from heptane (200 ml) to give mono-tert-butyl hexadecandioate, 15.9 g (33 %). Alternatively to recrystallization, the mono-ester can be purified by silica chromatography with ethyl acetate/heptane. ¹H-NMR (CDCl₃) δ: 2.35 (t, 2H), 2.20 (t, 2H), 1.65-1.55 (m, 4H), 1.44 (s, 9H), 1.34-1.20 (m, 20 H).

### 15-Isocyanatopentadecanoic acid tert-butyl ester

Mono-tert-butyl hexadecandioate (2.0 g, 5.8 mmol) was dissolved in tetrahydrofurane (50 mL). Triethylamine (2.04 mL, 14.6 mmol) was added and the mixture was cooled with ice and kept under nitrogen gas. Isocholorobutyl formate (1.6 mL, 11.7 mmol) was added dropwise over 15 mins, and the mixture was stirred on ice-bath for 1.5 hours. Sodium azide (6.5 g, 105 mmol) was added and the mixture was stirred on ice-bath 30 mins. Toluene (100 mL) was added and the organic phase was washed with water (2x50 mL) and dried over MgSO₄ and filtered. The mixture was heated on oil bath to 105 °C for 2 hours and then evaporated *in vacuo* to provide 15-isocyanatopentadecanoic acid tert-butyl ester (1.94, 98%).
LCMS: 287.3 Da (MH⁺ - tert-butyl)
¹H-NMR (CDCl₃) δ: 3.29 (t, 2H), 2.20 (t, 2H), 1.62-1.57 (m, 4H), 1.44 (s, 9H), 1.33-1.24 (m, 20 H).

### 15-Aminopentadecanoic acid tert-butyl ester

15-Isocyanatopentadecanoic acid tert-butyl ester (1.91 g, 5.6 mmol) was dissolved in tetrahydrofurane (20 mL), treated with 1 M aqueous NaOH (10 mL) and stirred at room temperature for 1 hour. Water (50 mL) was added and the mixture was extracted with ethyl acetate (2x 50 mL). The solution was dried over MgSO₄ and evaporated *in vacuo* to provide crude 15-amino-pentadecanoic acid tert-butyl ester (1.54 g, 88 %).
LCMS: 314.5 Da (MH⁺).

The crude product (1.26 g, 4.0 mmol) was dissolved in ether (90 mL) and treated with 4-toluene-sulfonic acid (688 mg, 3.6 mmol) dissolved in ether (25 mL). The mixture was stored at 5 °C overnight and the precipitate was collected by filtration and dried (1.1 g, 63 %). The tosylate (1.1 g, 2.3 mmol) was dissolved in ether, washed twice with 0.2 M Na₂CO₃, with water, dried over MgSO₄ and evaporated *in vacuo* to provide 15-aminopentadecanoic acid tert-butyl ester (630 mg, 92 %).

### 15-(4-Boronobenzenesulfonylamino)pentadecanoic acid tert-butyl ester

Lithium 4-sulfinylphenylboronic acid N-methyldiethanolamine ester (100 mg, 0.364 mmol; P. Vedsø, P.H. Ol,esen, T. Hoeg-Jensen, Synlett 2004, 892) was powdered by spatula, suspended in dichloromethane (2 mL), treated with N-chlorosuccinimide (49 mg, 0.364 mmol) and the mixture was stirred for 1 hour. *N,N*-Diisopropylethylamine (62 uL, 0.364 mmol) and 15-aminopentadecanoic acid tert-butyl ester (114 mg, 0.364 mmol) were added and the mixture was stirred overnight. The solvent was removed *in vacuo.* Ethyl acetate was added and the mixture was washed twice with 0.2 M HCl, water, brine, dried over MgSO₄, filtered and the solvent was removed *in vacuo* to provide 15-(4-boronobenzenesulfonylamino)pentadecanoic acid tert-butyl ester (152 mg, 84 %).
LCMS: 442.4 Da (MH⁺ - tert-butyl).

### 15-(4-Pinacolylboronobenzenesulfonylamino)pentadecanoic acid tert-butyl ester

15-(4-Boronobenzenesulfonylamino)pentadecanoic acid tert-butyl ester (152 mg, 0.306 mmol) was dissolved in ethyl acetate (3 mL), treated with pinacol (43 mg, 0.367 mmol) and MgSO₄ (44 mg, 0.367 mmol), and stirred for 30 mins. The mixture was washed twice with water, with brine, dried over MgSO₄, filtered and the solvent was removed *in vacuo* to provide 15-(4-pinacolylboronobenzenesulfonylamino)pentadecanoic acid tert-butyl ester (142 mg, 80 %).
¹H-NMR (CDCl₃) δ: 7.94 (d, 2H), 7.84 (d, 2H), 4.34 (t, 1H), 2.93 (m, 2H), 2.20 (t, 2H), 1.63-1.52 (m, 4H), 1.44 (s, 9H), 1.31-1.19 (m, 20 H).

### 15-(4-Pinacolylboronobenzenesulfonylamino)pentadecanoic acid

15-(4-Pinacolylboronobenzenesulfonylamino)pentadecanoic acid tert-butyl ester (142 mg, 0.245 mmol) was treated with trifluoroacetic acid (6 mL) for 30 minutes. The solvent was removed *in vacuo* to provide 15-(4-pinacolylboronobenzenesulfonylamino)pentadecanoic acid (128 mg, 100 %). ¹H-NMR (CDCl₃) δ: 7.94 (d, 2H), 7.83 (d, 2H), 6.72 (bs, 1H), 2.93 (t, 2H), 2.37 (t, 2H), 1.64 (m, 2H), 1.44 (m, 2H), 1.33-1.18 (m, 20 H).

### 15-(4-Pinacolylboronobenzenesulfonylamino)pentadecanoic acid N-hydroxysuccimide ester

15-(4-Pinacolylboronobenzenesulfonylamino)pentadecanoic acid (128 mg, 0.244 mmol) and N-hydroxysuccinimide (28 mg, 0.244 mmol) was dissolved in acetonitrile (1 mL), cooled with ice-bath and treated with *N,N*'-dicyclohexylcarbodiimide (50 mg, 0.244 mmol). The mixture was left overnight, filtered and the solvent was removed *in vacuo* to provide 15-(4-pinacolylboronobenzenesulfonylamino)pentadecanoic acid N-hydroxysuccimide ester (129 mg, 86%).
¹H-NMR (CDCl₃) δ: 7.93 (d, 2H), 7.83 (d, 2H), 4.36 (bs, 1H), 2.92 (t, 2H), 2.84 (s, 4H), 2.60 (t, 2H), 1.63-1.52 (m, 4H), 1.33-1.20 (m, 20 H).

### B29N^{ε}-15-(4-Boronobenzenesulfonylamino)pentadecanoyl desB30 human insulin

DesB30 human insulin (500 mg, 88 uM) was dissolved in 0.1 M Na₂CO₃ (5 mL) and treated with 15-(4-pinacolylboronobenzenesulfonylamino)pentadecanoic acid N-hydroxysuccimide ester (65 mg, 105 uM) in aceonitrile (5 mL). After 30 mins, pH was adjusted to 5.5 using 1 M HCl and the precipitate was collected by centrifugation. The product was purified by RP-HPLC on C18 column using buffer A: 0.1 % trifluoroacetic acid in water, buffer B: 0.1 % trifluoroacetic acid in acetonitrile, gradient 26 to 60 % B over 40 mins. The product pools were partially evaporated in vacuo and freeze dried providing B29*N*^{ε}-15-(4-boronobenzenesulfonylamino)pentadecanoyl desB30 human insulin (95 mg, 21 %).

LCMS: 1524.4 Da [M + 4H - 2x water]⁴⁺; buffer A: 0.1 % trifluoroacetic acid in water; buffer B: 0.1 % trifluoroacetic acid in acetonitrile, gradient 10 to 90 % B over 10 mins.

Purity by acidic HPLC: 99.3 %; C8 column, buffer A: 0.1 % trifluoroacetic acid in water; buffer B: 80 % acetonitrile/water, gradient 25 to 80 % B over 20 mins.

Purity by neutral HPLC: 99.5 %; C8 column, buffer A: 10 mM tris, 15 mM ammonium sulfate in 20 % acetonitrile/water.; buffer B: 80 % acetonitrile/water.-

The compounds in the following examples were prepared similarly as described above.

### Example 2

A22*N*^{ε}-16-(2,3-Difluoro-4-boronobenzoylamino)hexadecanoyl B29*N*^{ε}-16-(2,3-difluoro-4-borono-benzoylamino)hexadecanoyl A14E A22K B25H desB30 human insulin

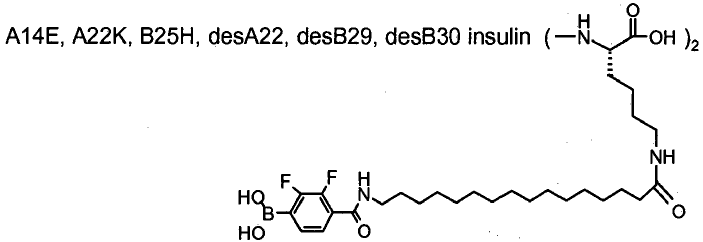

### Example 3

### B29N^{ε}-12-(3-Boronobenzenesulfonylamino)dodecanoyl desB30 human insulin

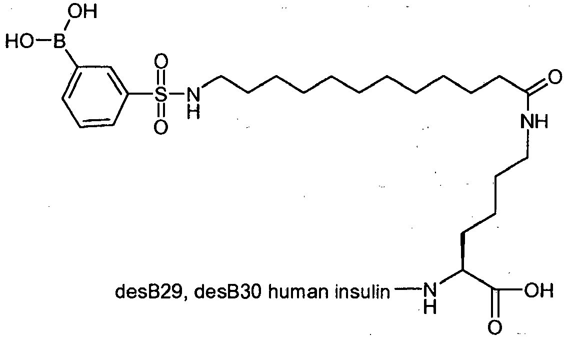

### Example 4

### B29N^{ε}-16-(2,3-Difluoro-4-boronobenzoylamino)hexadecanoyl-γ-L-glutamyl desB30 human insulin

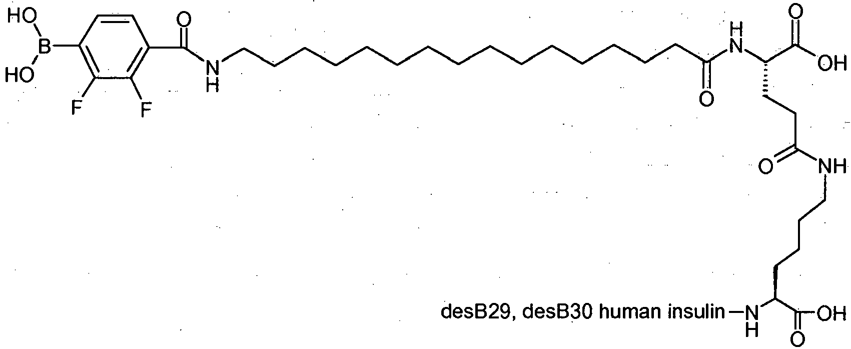

### Example 5

### B29N^{ε}-16-(3,5-Diboronobenzoylamino)hexadecanoyl desB30 human insulin

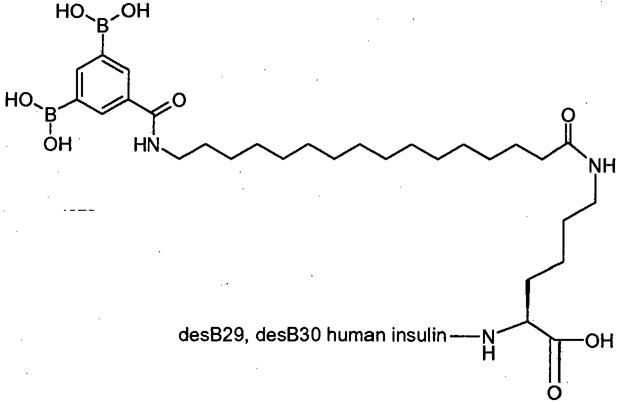

### Example 6

### B29N^{ε}-16-(2,3-Difluoro-4-boronobenzoylamino)hexadecanoyl desB30 human insulin

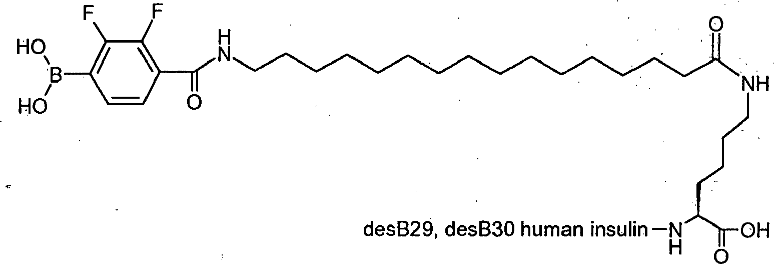

### Example 7

### B29N^{ε}-16-(3-Fluoro-4-boronobenzoylamino)hexadecanoyl desB30 human insulin

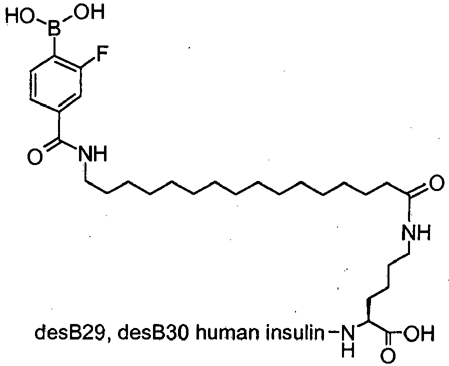

### Example 8

### B29N^{ε}-16-(3-Fluoro-4-boronobenzoylamino)hexadecanoyl-γ-L-glutamyl desB30 human insulin

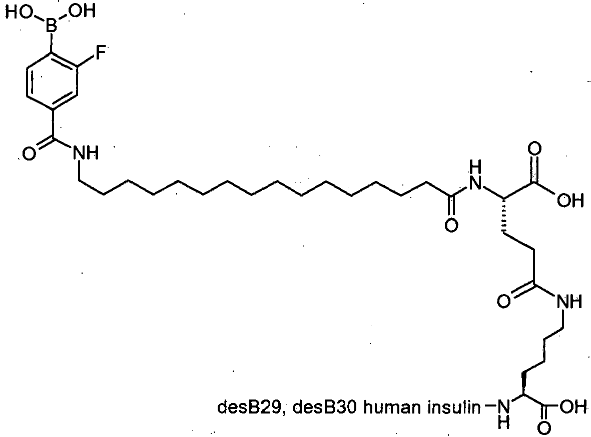

### Example 9

B29*N*^{ε}-16-(2,6-Difluoro-4-boronobenzoylamino)hexadecanonyl desB30 human insulin

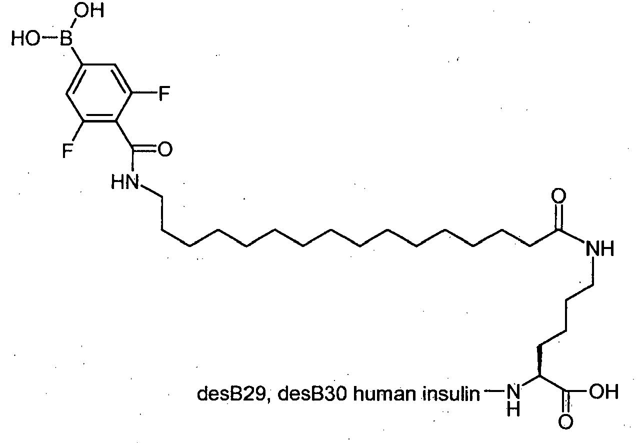

### Example 10

A1(*N,N*-Dimethyl), B1(*N,N*-dimethyl), B29*N*^{ε}-15-(4-boronobenzenesulfonylamino)pentadecanoyl desB30 human insulin

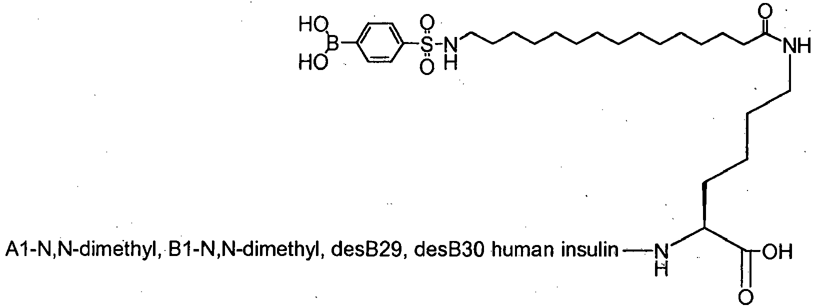

In this compound, the amino group present in A1Gly and present in B1Phe of the insulin moiety has been substituted by an *N,N-*dimethylamino group.

### Example 11

### A22N^{ε}-16-(3,5-Diboronobenzoylamino)hexadecanoyl A22K B29R desB30 human insulin

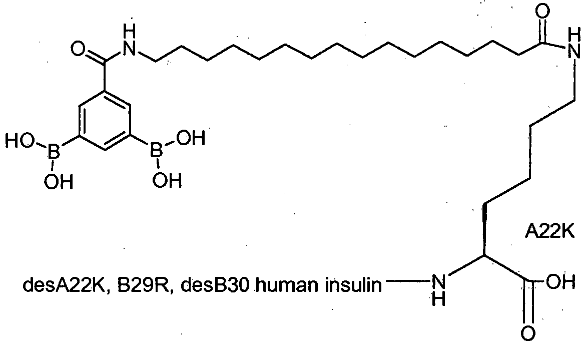

### Example 12

### A22N^{ε}-16-(3,5-Diboronobenzoylamino)hexadecanoyl A14E A22K B25H B29R desB30 human insulin

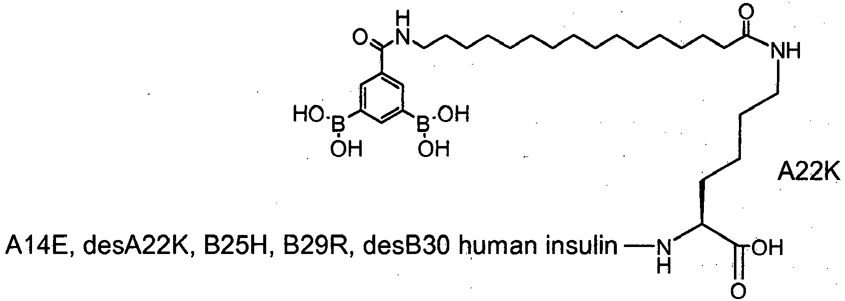

### Example 13

### A22N^{ε}-16-(3,5-Diboronobenzoylamino)hexadecanoyl-L-γ-glutamyl A14E A22K B25H B29R desB30 human insulin

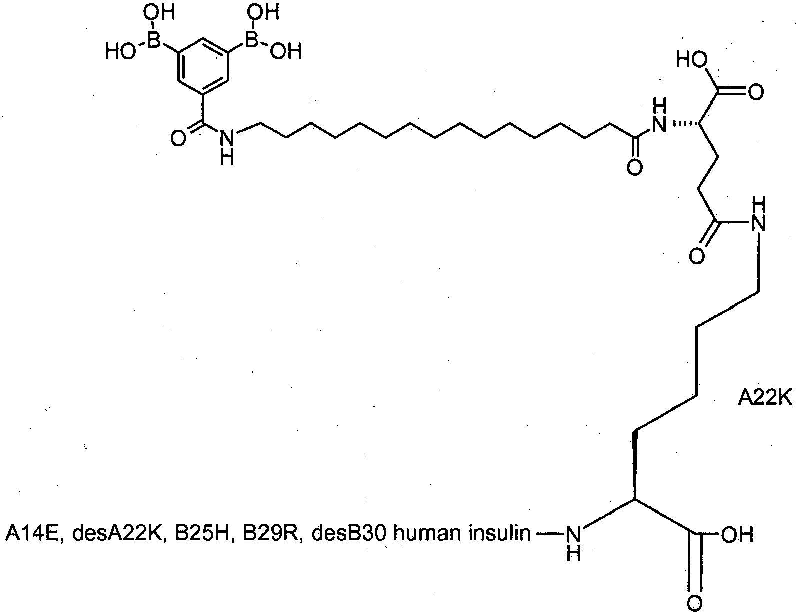

### Example 14

### B29N^{ε}-{4-[16-(3-Boronobenzenesulfonylamino)hexadecanoylsulfamoyl]butanoyl} desB30 human insulin

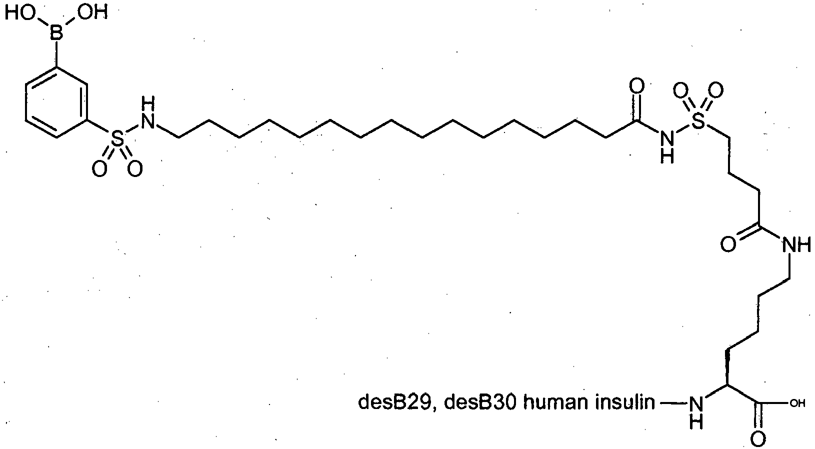

### Example 15

### A22N^{ε}-15-(3,5-Diboronobenzoylamino)pentadecanoyl B29N^{ε}-15-(3,5-diboronobenzoylamino)pentadecandioyl A14E A22K B25H desB30 human insulin

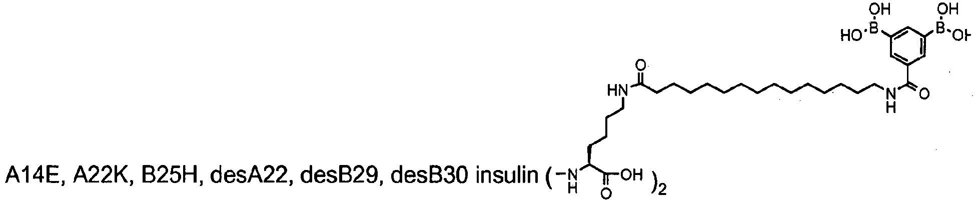

### Example 16

### A22N^{ε}-15-(3,5-Diboronobenzoylamino)pentadecanoyl B29N^{ε}-15-(3,5-diboronobenzoylamino)pentadecandioyl A22K desB30 human insulin

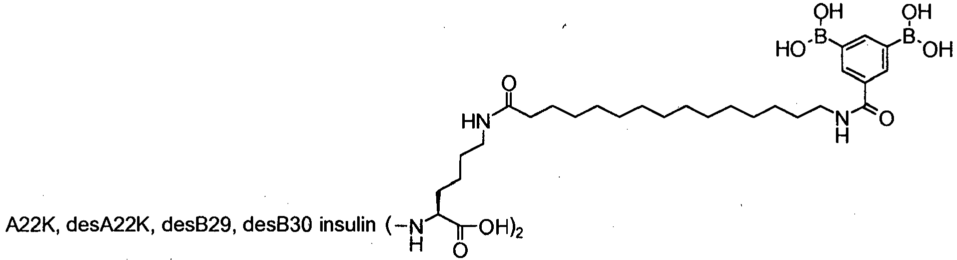

### Example 17

### B29N^{ε}-16-(3,5-Diboronobenzoylamino)hexadecanoyl-L-γ-glutamyl A14E B25H desB30 human insulin

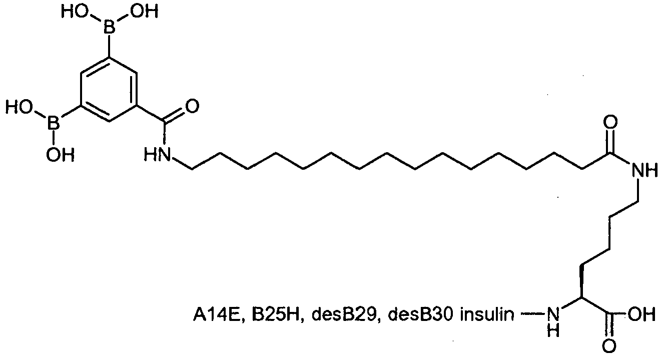

### Example 18

### A22N^{ε}-15-(3,5-Diboronobenzoylamino)pentadecanoyl-γ-L-glutamyl B29N^{ε}-15-(3,5-diboronobenzoylamino)pentadecandioyl-γ-L-glutamyl A14E A22K B25H desB30 human insulin

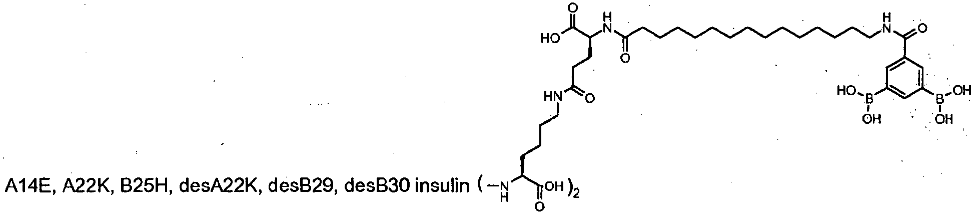

### Example 19

### B29N^{ε}-{4-[16-(3-Boronobenzenesulfonylamino)hexadecanoylsulfamoyl]butanoyl} A14E B25H desB30 human insulin

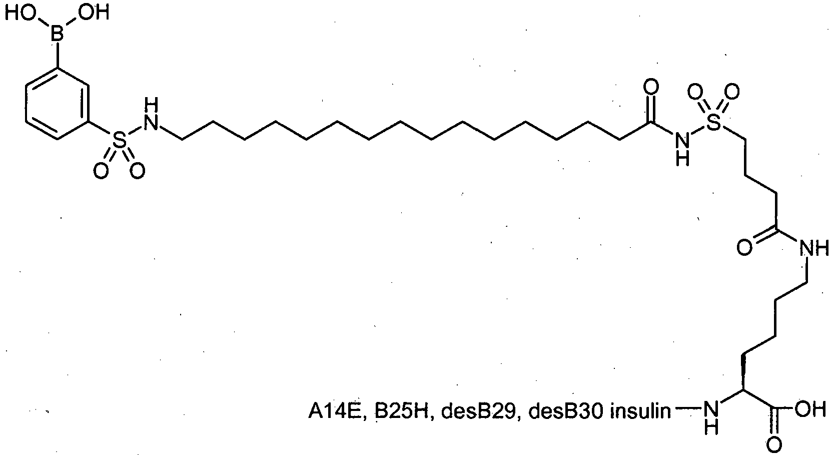

### Example 20

### A22N^{ε}-12-(3,5-Diboronobenzoylamino)dodecanoyl B29N^{ε}-12-(3,5-diboronobenzoylamino)dodecandioyl A14E A22K B25H desB30 human insulin

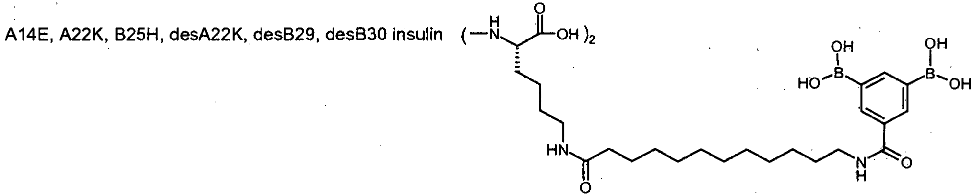

### Example 21

### A22N^{ε}-12-(3,5-Diboronobenzoylamino)dodecahoyl B29N^{ε}-12-(3,5-diboronobenzoylamino)-dodecandioyl A22K desB30 human insulin

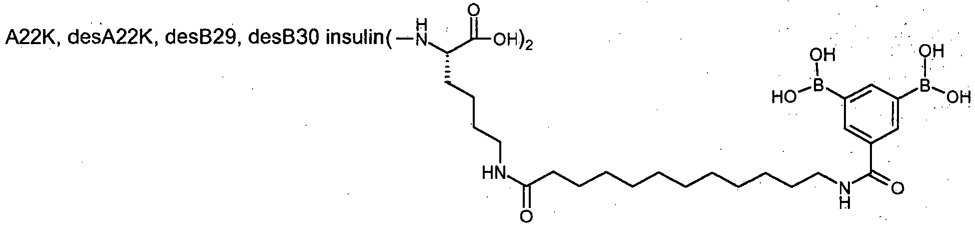

### Example 22

### B29N^{ε}-16-(2,3-Difluoro-4-boronobenzoylamino)hexadecanoyl A14E B25H des B30 human insulin

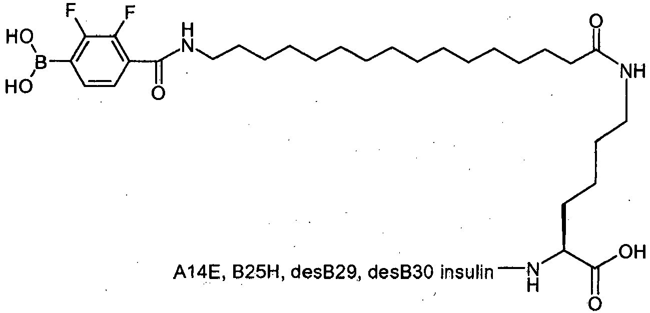

### Example 23

Albumin affinity of boronate fatty acid insulin derivatives towards human serum albumin can be evaluated by elution of the compounds through a chromatographic column carrying immobilized albumin (Chromtech Inc, HSA 50.3 column). The glucose sensitivity of insulin albumin binding can be determined by tuning glucose levels in the elution buffer and determine the shift in retention time as a function of the glucose concentration, table 1 below.
Buffer A: 10 mM phosphate, 2.7 mM KCI, 137 mM NaCl, pH 7.4 in water.
Buffer B: 10 mM phosphate, 2.7 mM KCI, 137 mM NaC!, pH 7.4 in water-iPrOH (1:1).
T 37 °C, flow 0.7 mL/min, 20 uL of 10 uM samples, UV monitor 220 nm.

| Gradient: time (minutes) | Percentage B |
|---|---|
| 0 | 0 |
| 5 | 40 |
| 15 | 50 |
| 16 | 0 |
| 20 | 0 |

The glucose affinity of the boronate building blocks can be measured in the competitive alizarin red sodium assay (Tetrahedron, 58, 5291-5300 (2002)).

The following table shows the retention times of some compounds of this invention with no glucose and with 50 mM glucose in the eluent. Compounds marked with an asterix were eluted with a gradient to 70 % buffer B.

| Example number | Δ Rt, minutes |
|---|---|
| 4 | 1.8 |
| 6 | 2.8 |
| 9 | 2.5 |
| 14 | 0.8 |
| 15 * | 1.0 |
| 16 * | 0.7 |
| 22 | 1.5 |

Herein, iPrOH designates isopropanol.

### Example 24

### B29N^{ε}-16-(2,3-Difluoro-4-hydroxybenzoylamino)hexadecanoyl A14E B25H desB30 human insulin

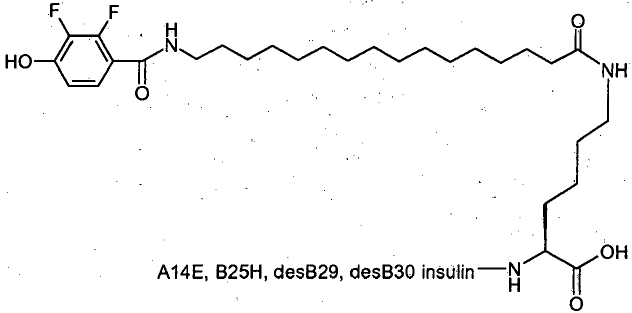

Insulin of example 22 (50 mg) was dissolved in aqueous hydrogen peroxide (500 mM, 5 mL) at room temperature and left for 20 hours. The insulin example 24 was isolated by isoprecipitation at pH 5.5 and purified by HPLC as described in example 1; LCMS [M⁴⁺] = 1519.0 Da.

### Example 25

### A22N^{ε}-12-(3,5-Diboronobenzoylamino)dodecanoyl-gamma-L-glutamyl B29N^{ε}-12-(3,5-diboronobenzoylamino)dodecandioyl-gamma-L-glutamyl A22K desB30 human insulin

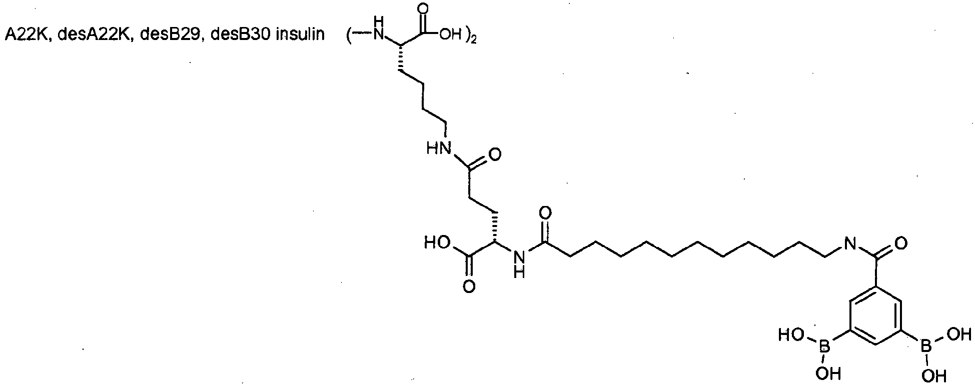

### Example 26

### A22N^{ε}-12-(3,5-Diboronobenzoylamino)dodecanoyl-gamma-L-glutamyl B29N^{ε}-12-(3,5-diboronobenzoylamino)dodecandioyl-gamma-L-glutamyl A14E A22K B25H desB30 human insulin

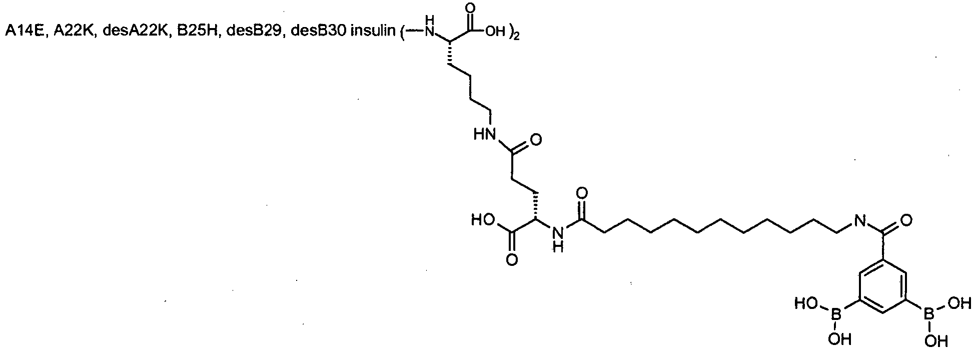

### Example 27

### B29N^{ε}-16-(2,6-Difluoro-4-boronobenzoylamino)pentadecanoyl A14E B25H desB30 human insulin

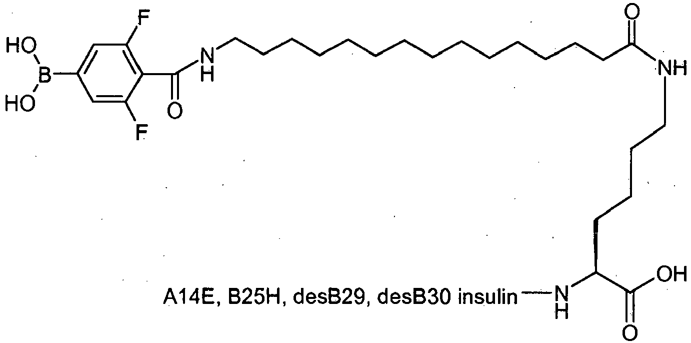

### Example 28

### B29N^{ε}-16-(2,6-Difluoro-4-boronobenzoylamino)hexadecanoyl A14E B25H desB30 human insulin

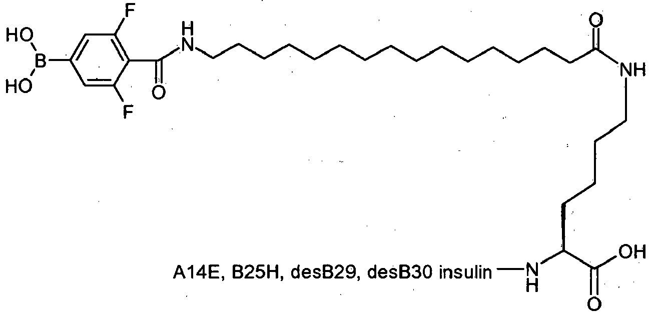

### Example 29

### B29N^{ε}-16-(4-Boronobenzoylamino)hexadecanoyl A14E B25H desB30 human insulin

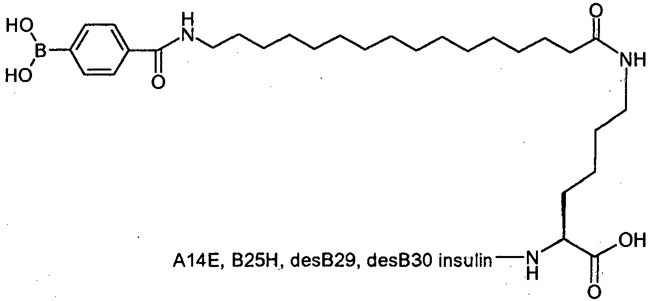

### Example 30

### I.v. bolus of a glucose-dependent insulin of example 22 or a non-glucose-dependent insulin of example 24 or vehicle in Wistar rats and ZDF rats (UIR100205-0143)

*Materials and Methods:* 30 male, fed Wistar rats (250 g) and 30 male, fed ZDF rats (350 g, 12 weeks old) were anesthetized using Hypnorm-Dormicum (0.081 mg/ml fentanyl citrate (VetaPharma Ltd.), 1.25 mg/ml Midazolam (Roche)) 2 ml/kg as a priming dose and additional 1 ml/kg to timepoint -5 min prior to test substance dosing, and then 1 ml/kg every 45 minutes (4 times). The Wistar rats show initial blood glucose level on 6-7 mmol/l whereas the ZDF rats show level on 20-25 mmol/l.

Both the Wistar rats and the ZDF rats were allocated into 5 groups, 6 rats in each (eg 10 groups in total). The animals were dosed with an intravenous injection in a tail vein (1 ml/kg) of either vehicle (5 mM phosphate buffer, 140 mM NaCl, 70 ppm polysorbate 20, pH 7.4) or insulin of example 22 (i.e., a compound according to the present invention) or insulin of example 24 (i.e., a compound corresponding to the compound of example 22 but lacking the -B(OH)₂ group). Two doses of each analogue were tested: 1.2 nmol/kg or 3.6 nmol/kg in Wistar rats, and 3.6 nmol/kg or 7.2 nmol/kg in ZDF rats. Blood samples of 100 µl (tail tip capillary puncture) for determination of plasma insulin were collected in microvette tubes at time 3, 15, 30, 60, 120, 180 and 240 minutes after insulin dosing.

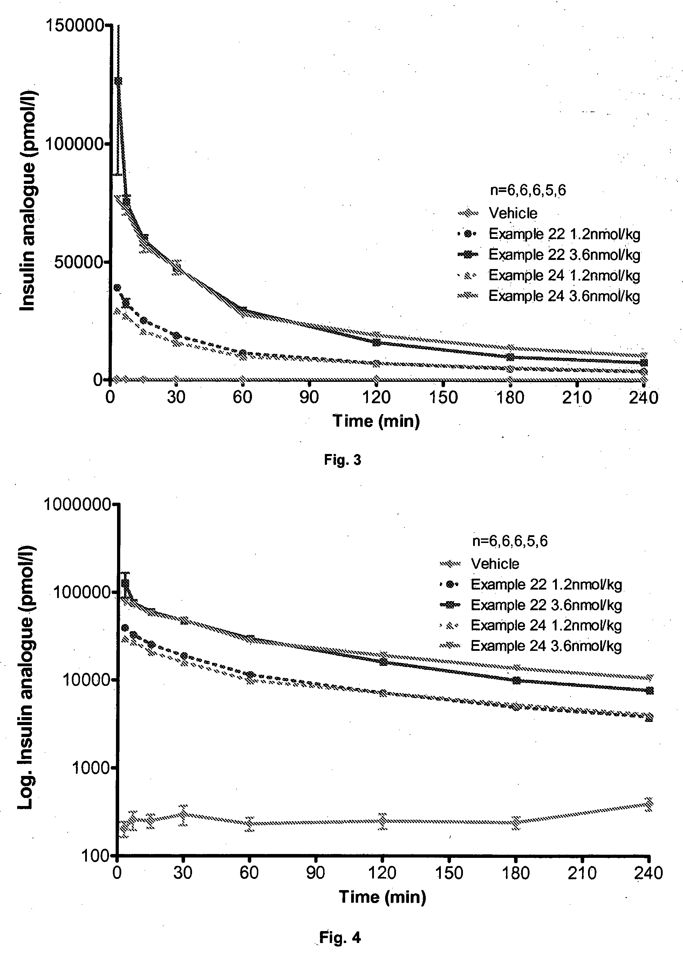

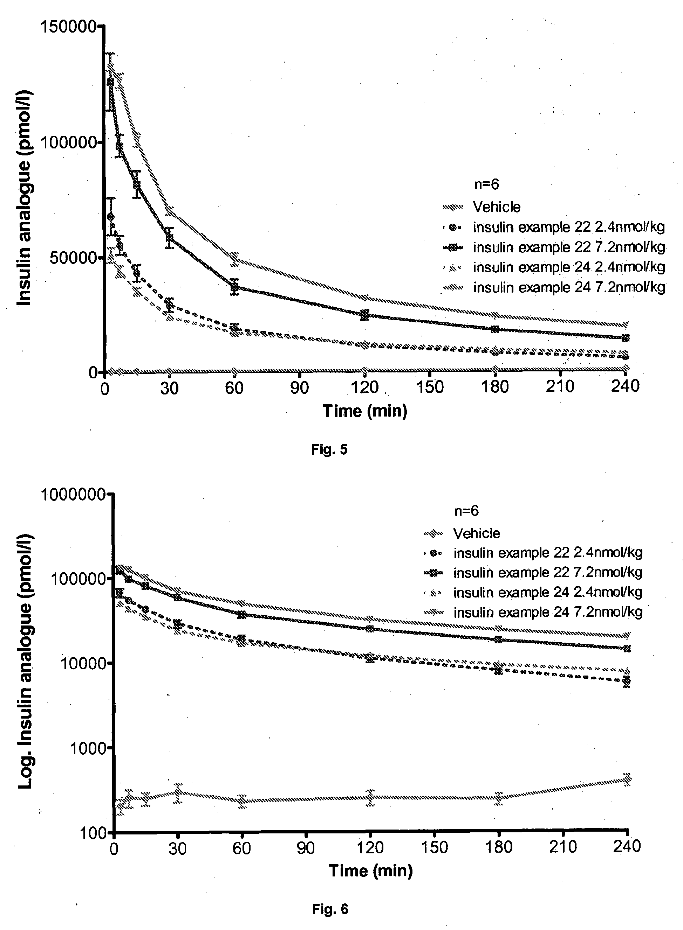

Figures 3-6 show the plasma-elimination profiles of the two analogues (each in two doses) after an i.v. bolus injection to Wistar rats and ZDF rats.

When pharmacokinetic parameters were estimated using the programme WinNonlin 5.2 (table below) insulin of example 22 has a mean residence time (MRT) in Wistar rats of 158 ± 42 min (low dose) and 121 ±10 min (high dose), whereas insulin of example 24 has MRT of 178 ± 22 min (low dose) and 175 ± 27 min (high dose). The corresponding values for ZDF rats were for insulin of example 22, 144 ±21 min and 179 ± 16 min and for insulin of example 24, 224 ± 24 min and 203 ±14 min. There is a statistical significant difference between insulin of example 22 and 24 when the high doses of these insulins are compared, both in Wistar rats and in ZDF rats (p<0.05 students t-test). For the low dose only significant differences were seen between insulin of example 22 and 24 in the ZDF rats (p<0.05). Corresponding significant differences were seen when elimination half-lives of insulin of example 22 and 24 are compared. The glucose-dependent insulin of example 22 (i.e., a compound according to the present invention) is thus eliminated faster than the non-glucose-dependent analogue insulin of example 24 under conditions of high blood glucose.

| Insulin example | Dose (nmol/kg) | | Vz (ml/kg) | CL (ml/kg/min) | MRT (min) | T½ (min) | Remarks |
|---|---|---|---|---|---|---|---|
| 22 | 1.2 | MEAN | 72 | 0.37 | 158 | 132 | n=6 |
| | | SD | 11 | 0.05 | 42 | 30 | Wistar |
| 22 | 2.4 | MEAN | 90 | 0.51 | 144 | 124 | n=6 |
| | | SD | 19 | 0.13 | 21 | 19 | ZDF |
| 22 | 3.6 | MEAN | 80 | 0.49 | 121 | 112 | n=6 |
| | | SD | 9 | 0.06 | 10 | 5 | Wistar |
| 22 | 7.2 | MEAN | 144 | 0.67 | 179 | 148 | n=6 |
| | | SD | 29 | 0.11 | 16 | 13 | ZDF |
| 24 | 1.2 | MEAN | 80 | 0.39 | 178 | 143 | n=5 |
| | | SD | 6 | 0.05 | 22 | 16 | Wistar |
| 24 | 2.4 | MEAN | 111 | 0.44 | 224 | 177 | n=6 |
| | | SD | 15 | 0.08 | 24 | 20 | ZDF |
| 24 | 3.6 | MEAN | 90 | 0.43 | 175 | 140 | n=6 |
| | | SD | 13 | 0.02 | 27 | 24 | Wistar |
| 24 | 7.2 | MEAN | 117 | 0.49 | 203 | 166 | n=6 |
| | | SD | 8 | 0.02 | 14 | 12 | ZDF |

| | | | | |
|---|---|---|---|---|
| T-test low dose Wistar | 0.13 | 0.52 | 0.33 | 0.64 |
| T-test low dose ZDF | 0.06 | 0.32 | 0.00 | 0:00 |
| T-test high dose Wistar | 0.20 | 0.05 | 0.00 | 0.02 |
| T-test high dose ZDF | 0.07 | 0.01 | 0.02 | 0.04 |

## Claims

1. An insulin derivative of the general formula I: (Z¹-Y¹-X¹)ₘ-Ins-(X-Y-Z)ₙ wherein n is zero or 1; m is zero or 1; with the proviso that not both n and m are zero; Ins represents insulin or an insulin analogue as defined herein from which a hydrogen atom has been removed from an ε amino group present in a B29 lysine residue and/or present in a A22 lysine residue and wherein the amino group present in the N terminal amino acid residue in the A and/or B chain, optionally, is substituted by one or two alkyl groups (preferably methyl or ethyl), wherein the group(s) of the general formula -X-Y-Z and/or -X¹-Y¹-Z¹ is/are attached to an ε amino group in a Lys (K) residue in the insulin molecule; X and X¹, independently of each other, is a bond, a moiety of the general formula -CO-(C1-C3-alkylene)-CH(COOH)-NH- wherein the amino group is connected to the Y/Y¹ moiety or a moiety of the general formula -CO-(C₁-C₃-alkylene)-SO₂-NH-, wherein the amino group is connected to the Y/Y¹ moiety; Y and Y¹, independently of each other, is ω-amino alkanoyl group containing 6 to 22 carbon atoms and wherein a hydrogen atom has been removed from the ω-amino group; and Z and Z¹, independently of each other, is a group of one of the following two, general formula -SO₂-Ar or -CO-Ar, wherein Ar is a phenyl group substituted by one or more -B(OH)₂ groups and, optionally, substituted by one or more halogen atoms.

2. The insulin derivative according to claim 1 having the general formula I: (Z¹-Y¹-X¹)ₘ-Ins-(X-Y-Z)ₙ wherein n is zero or 1; m is zero or 1; with the proviso that not both n and m are zero; Ins represents insulin or an insulin analogue as defined herein from which a hydrogen atom has been removed from an ε amino group present in a B29 lysine residue and/or present in a A22 lysine residue and wherein the amino group present in the N terminal amino acid residue in the A and/or B chain, optionally, is substituted by one or two alkyl groups (preferably methyl or ethyl), wherein the group(s) of the general formula -X-Y-Z and/or -X¹-Y¹-Z¹ is/are attached to an ε amino group in a Lys (K) residue in the insulin molecule; X and X¹, independently of each other, is a bond, a γ-glutamyl group wherein the amino group is connected to the Y/Y¹ moiety or a moiety of the general formula -CO-(C₁-C₃-alkylene)-SO₂-NH-, wherein the amino group is connected to the Y/Y¹ moiety; Y and Y¹, independently of each other, is ω-amino alkanoyl group containing 6 to 22 carbon atoms and wherein a hydrogen atom has been removed from the ω-amino group; and Z and Z¹, independently of each other, is a group of one of the following two, general formula -SO₂-A or -CO-Ar, wherein Ar is a phenyl group substituted by one or more -B(OH)₂ groups and, optionally, substituted by one or more halogen atoms.

3. A compound according to any one of the preceding claims, to the extend possible, selected from the group consisting of B29*N*^{ε}-15-(4-boronobenzenesulfonylamino)pentadecanoyl desB30 human insulin; A22*N*^{ε}-16-(2,3-difluoro-4-boronobenzoylamino)hexadecanoyl B29N(epsilon)-16-(2,3-difluoro-4-boronobenzoylamino)hexadecanoyl A14E A22K B25H desB30 human insulin; B29*N*^{ε}-12-(3-boronobenzenesulfonylamiho)dodecanoyl desB30 human insulin; B29*N*^{ε}-16-(2,3-difluoro-4-boronobenzoylamino)hexadecanoyl-γ-L-glutamyl desB30 human insulin; B29*N*^{ε}-16-(3,5-diboronobenzoylamino)hexadecanoyl desB30 human insulin; B29*N*^{ε}-16-(2,3-difluoro-4-boronobenzoylamino)hexadecanoyl desB30 human insulin; B29*N*^{ε}-16-(3-fluoro-4-boronobenzoylamino)hexadecanoyl desB30 human insulin; B29*N*^{ε}-16-(3-fluoro-4-boronobenzoylamino)hexadecanoyl-γ-L-glutamyl desB30 human insulin; B29*N*^{ε}-16-(2,6-difluoro-4-boronobenzoylamino)hexadecanonyl desB30 human insulin; A1(*N,N*-dimethyl), B1(*N,N*-dimethyl), B29*N*^{ε}-15-(4-boronobenzenesutfonylamino)pentadecanoyl desB30 human insulin; A22*N*^{ε}-16-(3,5-diboronobenzoylamino)hexadecanoyl A22K B29R desB30 human insulin; A22*N*^{ε}-16-(3,5-diboronobenzoylamino)hexadecanoyl A14E A22K B25H B29R desB30 human insulin; A22*N*^{ε}-16-(3,5-diboronobenzoylamino)hexadecanoyl-L-γ-glutamyl A14E A22K B25H B29R desB30 human insulin; B29-*N*^{ε}-{4-[16-(3-boronobenzenesulfonylamino)hexadecanoylsulfamoyl]butanoyl} desB30 human insulin; A22*N*^{ε}-15-(3,5-diboronobenzoylamino)pentadecanoyl B29*N*^{ε}-15-(3,5-diboronobenzoylamino)pentadecandioyl A14E A22K B25H desB30 human insulin; A22*N*^{ε}-15-(3,5-diboronobenzoylamino)pentadecanoyl B29*N*^{ε}-15-(3,5-diboronobenzoylamino)pentadecandioyl A22K desB30 human insulin; B29*N*^{ε}-16-(3,5-diboronobenzoylamino)hexadecanoyl-L-γ-glutamyl A14E B25H desB30 human insulin; A22*N*^{ε}-15-(3,5-diboronobenzoylamino)pentadecanoyl-γ-L-glutamyl B29*N*^{ε}-15-(3,5-diboronobenzoylamino)pentadecandioyl-γ-L-glutamyl A14E A22K B25H desB30 human insulin; B29*N*^{ε}-{4-[16-(3-boronobenzenesulfonylamino)hexadecanoylsulfamoyl]butanoyl} A14E B25H desB30 human insulin; A22*N*^{ε}-12-(3,5-diboronobenzoylamino)dodecanoyl B29*N*^{ε}-12-(3,5-diboronobenzoylamino)dodecandioyl A14E A22K B25H desB30 human insulin; A22*N*^{ε}-12-(3,5-diboronobenzoylamino)dodecanoyl B29*N*^{ε}-12-(3,5-diboronobenzoylamino)dodecandioyl A22K desB30 human insulin; B29*N*^{ε}-16-(2,3-difluoro-4-boronobenzoylamino)hexadecanoyl A14E B25H des B30 human insulin; A22*N*^{ε}-12-(3,5-diboronobenzoylamino)dodecanoyl-gamma-L-glutamyl B29*N*^{ε}-12-(3,5-diboronobenzoylamino)-dodecandioyl-gamma-L-glutamyl A22K desB30 human insulin; A22*N*^{ε}-12-(3,5-diboronobenzoylamino)dodecanoyl-gamma-L-glutamyl B29*N*^{ε}-12-(3,5-diboronobenzoylamino)dodecandioyl-gamma-L-glutamyl A14E A22K B25H desB30 human insulin; B29*N*^{ε}-16-(2,6-difluoro-4-borono-benzoylamino)pentadecanoyl A14E B25H desB30 human insulin; B29*N*^{ε}-16-(2,6-difluoro-4-boronobenzoylamino)hexadecanoyl A14E B25H desB30 human insulin; and B29*N*^{ε}-16-(4-borono-benzoylamino)hexadecanoyl A14E B25H desB30 human insulin.

4. An insulin derivative according to any one of the preceding claims for use as a medicament.

5. An insulin derivative according to any one of the preceding product claims for use in the treatment of diabetes.

## Patentansprüche

1. Insulinderivat der allgemeinen Formel I_{:} (Z¹-Y¹-X¹)ₘ-Ins-(X-Y-Z)ₙ, wobei n gleich null oder 1 ist; m gleich null oder 1 ist; mit der Maßgabe, dass nicht sowohl n als auch m gleich null sind; Ins Insulin oder ein Insulinanalog mit der vorliegend angegebenen Bedeutung repräsentiert, aus dem ein Wasserstoffatom aus einer in einem B29-Lysinrest und/oder in einem A22-Lysinrest vorliegenden ε-Aminogruppe entfernt wurde, und wobei die im N-terminalen Aminosäurerest in der A- und/oder B-Kette vorliegende Aminogruppe gegebenenfalls mit einer oder zwei Alkylgruppen (vorzugsweise Methyl oder Ethyl) substituiert ist, wobei die Gruppe(n) der allgemeinen Formel -X-Y-Z und/oder -X¹-Y¹-Z¹ an eine ε-Aminogruppe in einem Lys(K)-Rest im Insulinmolekül gebunden ist/sind; X und X¹ unabhängig voneinander für eine Bindung, eine Gruppierung der allgemeinen Formel -CO-(C1-C3-Alkylen)-CH(COOH)-NH-, wobei die Aminogruppe mit der Y/Y¹-Gruppierung verbunden ist, oder eine Gruppierung der allgemeinen Formel -CO-(C₁-C₃-Alkylen)-SO₂-NH-, wobei die Aminogruppe mit der Y/Y¹-Grup-pierung verbunden ist, stehen; Y und Y¹ unabhängig voneinander für eine ω-Aminoalkanoylgruppe mit 6 bis 22 Kohlenstoffatomen, und wobei ein Wasserstoffatom aus der ω-Aminogruppe entfernt wurde, stehen; und Z und Z¹ unabhängig voneinander für eine Gruppe einer der beiden folgenden allgemeinen Formeln, -SO₂-Ar bzw. -CO-Ar, stehen, wobei Ar für eine mit einer oder mehreren -B(OH)₂-Gruppen und gegebenenfalls mit einem oder mehreren Halogen-atomen substituierte Phenylgruppe steht.

2. Insulinderivat nach Anspruch 1 mit der allgemeinen Formel I: (Z¹-Y¹-X¹)ₘ-Ins-(X-Y-Z)ₙ, wobei n gleich null oder 1 ist; m gleich null oder 1 ist; mit der Maßgabe, dass nicht sowohl n als auch m gleich null sind; Ins Insulin oder ein Insulinanalog mit der vorliegend angegebenen Bedeutung repräsentiert, aus dem ein Wasserstoffatom aus einer in einem B29-Lysinrest und/oder in einem A22-Lysinrest vorliegenden ε-Aminogruppe entfernt wurde, und wobei die im N-terminalen Aminosäurerest in der A- und/oder B-Kette vorliegende Aminogruppe gegebenenfalls mit einer oder zwei Alkylgruppen (vorzugsweise Methyl oder Ethyl) substituiert ist, wobei die Gruppe(n) der allgemeinen Formel -X-Y-Z und/oder -X¹-Y¹-Z¹ an eine ε-Aminogruppe in einem Lys(K)-Rest im Insulinmolekül gebunden ist/sind; X und X¹ unabhängig voneinander für eine Bindung, eine γ-Glutamylgruppe, wobei die Aminogruppe mit der Y/Y¹-Gruppierung verbunden ist, oder eine Gruppierung der allgemeinen Formel -CO-(C₁-C₃-Alkylen)-SO₂-NH-, wobei die Aminogruppe mit der Y/Y¹-Grup-pierung verbunden ist, stehen; Y und Y¹ unabhängig voneinander für eine ω-Aminoalkanoylgruppe mit 6 bis 22 Kohlenstoffatomen, und wobei ein Wasserstoffatom aus der ω-Aminogruppe entfernt wurde, stehen; und Z und Z¹ unabhängig voneinander für eine Gruppe einer der beiden folgenden allgemeinen Formeln, -SO₂-Ar bzw. -CO-Ar, stehen, wobei Ar für eine mit einer oder mehreren -B(OH)2-Gruppen und gegebenenfalls mit einem oder mehreren Halogen-atomen substituierte Phenylgruppe steht.

3. Verbindung nach einem der vorhergehenden Ansprüche, soweit möglich, ausgewählt aus der Gruppe bestehend aus B29*N*^{ε}-15-(4-Boronobenzol-sulfonylamino)pentadecanoyl-desB30-Humaninsulin; A22*N*^{ε}-16-(2,3-Difluor-4-boronobenzoylamino)hexadecanoyl-B29N(epsilon)-16-(2,3-difluor-4-boronobenzoylamino)hexadecanoyl-A14E-A22K-B25H-desB30-Humaninsulin; B29*N*^{ε}-12-(3-Boronobenzolsulfonylamino)dodecanoyl-desB30-Humaninsulin; B29*N*^{ε}-16-(2,3-Difluor-4-boronobenzoylamino)hexadecanoyl-γ-L-glutamyl-desB30-Humaninsulin; B29*N*^{ε}-16-(3,5-Diboronobenzoylamino)hexadecanoyl-desB30-Humaninsulin; B29*N*^{ε}-16-(2,3-Difluor-4-boronobenzoylamino)hexadecanoyl-desB30-Humaninsulin; B29*N*^{ε}-16-(3-Fluor-4-boronobenzoylamino)hexadecanoyl-desB30-Humaninsulin; B29*N*^{ε}-16-(3-Fluor-4-boronobenzoylamino)hexadecanoyl-γ-L-glutamyl-desB30-Humaninsulin; B29*N*^{ε}-16-(2,6-Difluor-4-boronobenzoylamino)hexadecanoyl-desB30-Humaninsulin; A1 (*N,N-*Dimethyl),B1(*N,N*-dimethyl),B29*N*^{ε}-15-(4-Boronobenzolsulfonylamino)pentadecanoyl-desB30-Humaninsulin; A22*N*^{ε}-16-(3,5-Diboronobenzoylamino)hexadecanoyl-A22K-B29R-desB30-Humaninsulin; A22*N*^{ε}-16-(3,5-Diboronobenzoylamino)hexadecanoyl-A14E-A22K-B25H-B29R-desB30-Humaninsulin; A22*N*^{ε}-16-(3,5-Diboronobenzoylamino)hexadecanoyl-L-γ-glutamyl-A14E-A22K-B25H-B29R-desB30-Humaninsulin; B29-*N*^{ε}-{4-[16-(3-Boronobenzolsulfonylamino)hexadecanoyl-sulfamoyl]butanoyl}-desB30-Humaninsulin; A22*N*^{ε}-15-(3,5-Diboronobenzoylamino)pentadecanoyl-B29*N*^{ε}-15-(3,5-diboronobenzoylamino)pentadecandioyl-A14E-A22K-B25H-desB30-Humaninsulin; A22*N*^{ε}-15-(3,5-Diboronobenzoylamino)pentadecanoyl-B29*N*^{ε}-15-(3,5-diboronobenzoylamino)pentadecandioyl-A22K-desB30-Humaninsulin; B29*N*^{ε}-16-(3,5-Diboronobenzoylamino)-hexadecanoyl-L-γ-glutamyl-A14E-B25H-desB30-Humaninsulin; A22*N*^{ε}-15-(3,5-Diboronobenzoylamino)pentadecanoyl-γ-L-glutamyl-B29*N*^{ε}-15-(3,5-diboronobenzoylamino)pentadecandioyl-γ-L-glutamyl-A14E-A22K-B25H-desB30-Humaninsulin; B29*N*^{ε}-{4-[16-(3-Boronobenzolsulfonylamino)hexadecanoylsulfamoyl]-butanoyl}-A14E-B25H-desB30-Humaninsulin; A22*N*^{ε}-12-(3,5-Diboronobenzoylamino)dodecanoyl-B29*N*^{ε}-12-(3,5-diboronobenzoylamino)dodecandioyl-A14E-A22K-B25H-desB30-Humaninsulin; A22*N*^{ε}-12-(3,5-Diboronobenzoylamino)dodecanoyl-B29*N*^{ε}-12-(3,5-diboronobenzoylamino)dodecandioyl-A22K-desB30-Humaninsulin; B29*N*^{ε}-16-(2,3-Difluor-4-boronobenzoylamino)hexadecanoyl-A14E-B25H-desB30-Humaninsulin; A22*N*^{ε}-12-(3,5-Diboronobenzoylamino)dodecanoyl-gamma-L-glutamyl-B29*N*^{ε}-12-(3,5-diboronobenzoylamino)dodecandioylgamma-L-glutamyl-A22K-desB30-Humaninsulin; A22*N*^{ε}-12-(3,5-Diboronobenzoylamino)dodecanoyl-gamma-L-glutamyl-B29*N*^{ε}-12-(3,5-diboronobenzoylamino)-dodecandioyl-gamma-L-glutamyl-A14E-A22K-B25H-desB30-Humaninsulin; B29*N*^{ε}-16-(2,6-Difluor-4-boronobenzoylamino)pentadecanoyl-A14E-B25H-desB30-Humaninsulin; B29*N*^{ε}-16-(2,6-Difluor-4-boronobenzoylamino)hexadecanoyl-A14E-B25H-desB30-Humaninsulin; und B29*N*^{ε}-16-(4-Boronobenzoylamino)hexadecanoyl-A14E-B25H-desB30-Humaninsulin.

4. Insulinderivat nach einem der vorhergehenden Ansprüche zur Verwendung als Arzneimittel.

5. Insulinderivat nach einem der vorhergehenden Produktansprüche zur Verwendung bei der Behandlung von Diabetes.

## Revendications

1. Dérivé d'insuline de formule générale I :
(Z¹-Y¹-X¹)ₘ-Ins-(X-Y-Z)ₙ
dans laquelle n vaut zéro ou 1 ; m vaut zéro ou 1 ; sous réserve que n et m ne valent pas tous deux zéro ; Ins représente l'insuline ou un analogue d'insuline tel que défini ici dont un atome d'hydrogène a été retiré d'un groupe ε-amino présent dans un résidu de lysine en B29 et/ou présent dans un résidu de lysine en A22 et dont le groupe amino présent dans le résidu d'acide aminé N-terminal de la chaîne A et/ou B est éventuellement substitué par un ou deux groupes alkyle (de préférence méthyle ou éthyle), et dans laquelle le ou les groupes de formules générales -X-Y-Z et/ou -X¹-Y¹-Z¹ sont rattachés à un groupe ε-amino dans un résidu Lys (K) de la molécule d'insuline ; X et X¹, indépendamment l'un de l'autre, sont une liaison, un fragment de formule générale -CO-(alkylène en C₁ à C₃-CH(COOH)-NH- dans lequel le groupe amino est connecté au fragment Y/Y¹ ou un fragment de formule générale -CO-(alkylène en C₁ à C₃)-SO₂-NH- dans lequel le groupe amino est connecté au fragment Y/Y¹ ; Y et Y¹, indépendamment l'un de l'autre, sont un groupe ω-aminoalcanoyle contenant 6 à 22 atomes de carbone et dont un atome d'hydrogène a été retiré du groupe ω-amino ; et Z et Z¹, indépendamment l'un de l'autre, sont un groupe répondant à l'une des deux formules générales suivantes : -SO₂-Ar et -CO-Ar, dans lesquelles Ar est un groupe phényle substitué par un ou plusieurs groupes -B(OH)₂ et éventuellement substitué par un ou plusieurs atomes d'halogène.

2. Dérivé d'insuline selon la revendication 1 de formule générale I : (Z¹-Y¹-X¹)ₘ-Ins-(X-Y-Z)ₙ dans laquelle n vaut zéro ou 1 ; m vaut zéro ou 1 ; sous réserve que n et m ne valent pas tous deux zéro ; Ins représente l'insuline ou un analogue d'insuline tel que défini ici dont un atome d'hydrogène a été retiré d'un groupe ε-amino présent dans un résidu de lysine en B29 et/ou présent dans un résidu de lysine en A22 et dont le groupe amino présent dans le résidu d'acide aminé N-terminal de la chaîne A et/ou B est éventuellement substitué par un ou deux groupes alkyle (de préférence méthyle ou éthyle), et dans laquelle le ou les groupes de formules générales -X-Y-Z et/ou -X¹-Y¹-Z¹ sont rattachés à un groupe ε-amino dans un résidu Lys (K) de la molécule d'insuline ; X et X¹, indépendamment l'un de l'autre, sont une liaison, un groupe γ-glutamyle dans lequel le groupe amino est connecté au fragment Y/Y¹ ou un fragment de formule générale -CO-(alkylène en C₁ à C₃)-SO₂-NH- dans lequel le groupe amino est connecté au fragment Y/Y¹ ; Y et Y¹, indépendamment l'un de l'autre, sont un groupe ω-aminoalcanoyle contenant 6 à 22 atomes de carbone et dont un atome d'hydrogène a été retiré du groupe ω-amino ; et Z et Z¹, indépendamment l'un de l'autre, sont un groupe répondant à l'une des deux formules générales suivantes : -SO₂-Ar et -CO-Ar, dans lesquelles Ar est un groupe phényle substitué par un ou plusieurs groupes -B(OH)₂ et éventuellement substitué par un ou plusieurs atomes d'halogène.

3. Composé selon l'une quelconque des revendications précédentes, dans la mesure du possible, choisi dans l'ensemble constitué par la B29*N*^{ε}-15-(4-boronobenzènesulfonylamino)pentadécanoyl-desB30-insuline humaine ; l'A22*N*^{ε}-16-(2,3-difluoro-4-boronobenzoylamino)hexadécanoyl-B29N(epsilon)-16-(2,3-difluoro-4-boronobenzoylamino)hexadécanoyl-A14E-A22K-B25H-desB30-insuline humaine ; la B29*N*^{ε}-12-(3-boronobenzènesulfonylamino)dodécanoyl-desB30-insuline humaine ; la B29*N*^{ε}-16-(2,3-difluoro-4-boronobenzoylamino)hexadécanoyl-γ-L-glutamyl-desB30-insuline humaine ; la B29*N*^{ε}-16-(3,5-diboronobenzoylamino)hexadécanoyl-desB30-insuline humaine ; la B29*N*^{ε}-16-(2,3-difluoro-4-boronobenzoylamino)hexadécanoyl-desB30-insuline humaine ; la B29*N*^{ε}-16-(3-fluoro-4-boronobenzoylamino)hexadécanoyl-desB30-insuline humaine ; la B29*N*^{ε}-16-(3-fluoro-4-boronobenzoylamino)hexadécanoyl-γ-L-glutamyl-desB30-insuline humaine ; la B29*N*^{ε}-16-(2,6-difluoro-4-boronobenzoylamino)hexadécanoyl-desB30-insuline humaine ; l'A1(*N,N*-diméthyl), B1(*N,N*-diméthyl), B29*N*^{ε}-15-(4-boronobenzènesulfonylamino)pentadécanoyl-desB30-insuline humaine ; l'A22*N*^{ε}-16-(3,5-diboronobenzoylamino)hexadécanoyl-A22K-B29R-desB30-insuline humaine ; l'A22*N*^{ε}-16-(3,5-diboronobenzoylamino)hexadécanoyl-A14E-A22K-B25H-B29R-desB30-insuline humaine ; l'A22*N*^{ε}-16-(3,5-diboronobenzoylamino)hexadécanoyl-L-γ-glutamyl-A14E-A22K-B25H-B29R-desB30-insuline humaine ; la B29-*N*^{ε}-{4-[16-(3-boronobenzènesulfonylamino)hexadécanoyl-sulfamoyl]butanoyl}desB30-insuline humaine ; l'A22*N*^{ε}-15-(3,5-diboronobenzoylamino)pentadécanoyl-B29*N*^{ε}-15-(3,5-diboronobenzoylamino)pentadécanedioyl-A14E-A22K-B25H-desB30-insuline humaine ; l'A22*N*^{ε}-15-(3,5-diboronobenzoylamino)pentadécanoyl-B29*N*^{ε}-15-(3,5-diboronobenzoylamino)pentadécanedioyl-A22K-desB30-insuline humaine ; la B29*N*^{ε}-16-(3,5-diboronobenzoylamino)hexadécanoyl-L-γ-glutamyl-A14E-B25H-desB30-insuline humaine ; l'A22*N*^{ε}-15-(3,5-diboronobenzoylamino)pentadécanoyl-γ-L-glutamyl-B29*N*^{ε}-15-(3,5-diboronobenzoylamino)pentadécanedioyl-γ-L-glutamyl-A14E-A22K-B25H-desB30-insuline humaine ; la B29*N*^{ε}-{4-[16-(3-boronobenzènesulfonylamino)hexadécanoylsulfamoyl]butanoyl}-A14E-B25H-desB30-insuline humaine ; l'A22*N*^{ε}-12-(3,5-diboronobenzoylamino)dodécanoyl-B29*N*^{ε}-12-(3,5-diboronobenzoylamino)dodécanedioyl-A14E-A22K-B25H-desB30-insuline humaine ; l'A22*N*^{ε}-12-(3,5-diboronobenzoylamino)dodécanoyl-B29*N*^{ε}-12-(3,5-diboronobenzoylamino)dodécanedioyl-A22K-desB30-insuline humaine ; la B29*N*^{ε}-16-(2,3-difluoro-4-boronobenzoylamino)hexadécanoyl-A14E-B25H-desB30-insuline humaine ; l'A22*N*^{ε}-12-(3,5-diboronobenzoylamino)dodécanoyl-gamma-L-glutamyl-B29*N*^{ε}-12-(3,5-diboronobenzoylamino)dodécanedioyl-gamma-L-glutamyl-A22K-desB30-insuline humaine ; l'A22*N*^{ε}-12-(3,5-diboronobenzoylamino)dodécanoyl-gamma-L-glutamyl-B29*N*^{ε}-12-(3,5-diboronobenzoylamino)dodécanedioyl-gamma-L-glutamyl-A14E-A22K-B25H-desB30-insuline humaine ; la B29*N*^{ε}-16-(2,6-difluoro-4-boronobenzoylamino)pentadécanoyl-A14E-B25H-desB30-insuline humaine ; la B29*N*^{ε}-16-(2,6-difluoro-4-boronobenzoylamino)hexadécanoyl-A14E-B25H-desB30-insuline humaine ; et la B29*N*^{ε}-16-(4-boronobenzoylamino)hexadécanoyl-A14E-B25H-desB30-insuline humaine.

4. Dérivé d'insuline selon l'une quelconque des revendications précédentes, pour utilisation en tant que médicament.

5. Dérivé d'insuline selon l'une quelconque des revendications précédentes, pour utilisation dans le traitement du diabète.
